(19) Europäisches Patentamt
European Patent Office
Office européen des brevets

(11) Veröffentlichungsnummer: **0 244 627**
**A2**

(12) # EUROPÄISCHE PATENTANMELDUNG

(21) Anmeldenummer: 87104653.8

(22) Anmeldetag: 30.03.87

(51) Int. Cl.⁴: C07K 7/10 , C12N 15/00 , C12N 1/20 , A61K 37/64 , C12P 21/02

Die Bezeichnung der Erfindung wurde geändert (Richtlinien für die Prüfung im EPA, A-III, 7.3).

(30) Priorität: 10.04.86 GB 8608706

(43) Veröffentlichungstag der Anmeldung: 11.11.87 Patentblatt 87/46

(84) Benannte Vertragsstaaten: AT BE CH DE ES FR GB IT LI NL SE

(71) Anmelder: BAYER AG
Konzernverwaltung RP Patentabteilung
D-5090 Leverkusen 1 Bayerwerk(DE)

(72) Erfinder: Wilcken-Bergmann, Brigitte, Dr. v.
Mauenheimer Strasse 70
D-5000 Köln 60(DE)
Erfinder: Auerswald, Ernst-August, Dr.
Steinberger Weg 40
D-5600 Wuppertal 11(DE)
Erfinder: Müller-Hill, Benno, Prof. Dr.
Händelstrasse 53
D-500 Köln 1(DE)

(54) **Expressionsvektoren zur Gewinnung von Polypeptiden.**

(57) Die Erfindung betrifft Proteine und Polypeptide, pharmazeutische Zubereitungen, Promotoren, Operatoren, Gene, Vektoren, Wirtsorganismen und Verfahren zu deren Herstellung.

Die Erfindung betrifft Proteine und Polypeptide, insbesondere Met-Aprotinin und Met-Aprotinin-Homologe, deren Gewinnung über die rekombinante DNA-Technologie, erfolgt, Expressionsgene eines neuen Organismus, neue Expressionsvektoren einschließlich Promotoren, Operatoren, amplifizierte Gene und ein Verfahren zur Konstruktion von oligomeren Genen.

Die chemisch synthetisierten DNA-Moleküle, wie sie hier offenbart werden, sind durch ihre DNA-Sequenz charakterisiert.

EP 0 244 627 A2

# Expressionsvektoren zur Gewinnung von Polypeptiden, Verfahren zur verbesserten Expression von Polypeptiden, Wirtszellen, die die Expressionsvektoren enthalten, sowie die dadurch hergestellten Produkte

Die Erfindung betrifft Proteine und Polypeptide, pharmazeutische Zubereitungen, Promotoren, Operatoren, Gene, Vektoren, Wirtsorganismen und Verfahren zu deren Herstellung.

Die Erfindung betrifft Proteine und Polypeptide, insbesondere Met-Aprotinin und Met-Aprotinin-Homologe, deren Gewinnung über die rekombinante DNA-Technologie, erfolgt, Expressionsgene eines neuen Organismus, neue Expressionsvektoren einschließlich Promotoren, Operatoren, amplifizierte Gene und ein Verfahren zur Konstruktion von oligomeren Genen.

Die chemisch synthetisierten DNA-Moleküle, wie sie hier offenbart werden, sind durch ihre DNA-Sequenz charakterisiert.

Im Falle von Genen für Met-Aprotinin und Met-Aprotinin-Homologen kodiert die DNA-Sequenz für neue Polypeptide oder Polypeptide, die im wesentlichen in ihrer Aminosäuresequenz und Zusammensetzung mit der von Aprotinin oder Aprotinin-Homologen übereinstimmen und die biologische Aktivität von Aprotinin oder Aprotinin-Homologen besitzen.

Aprotinin ist ein allgemein bekanntes Peptid mit 58 Aminosäuren, das eine Inhibierung von Trypsin, Chymotrypsin, Plasmin und Kallikrein bewirkt. Es ist ein basischer Proteinasen-Inhibitor aus Organen des Rinds; Aprotinin, bezeichnet als Trasylol®, stellt ein wertvolles Arzneimittel zur Behandlung verschiedener Erkrankungen, wie z. B. hyperfibrinolytische Hämorrhagie und traumatischer-hämorrhagischer-Schock dar (s. H. Fritz und G. Wunderer, 1983, Drug Res. 33, 479 - 494).

Es konnte kürzlich gezeigt werden, daß Homologe von Aprotinin mit anderen Aminosäuren in der Position 15, anstelle von Lysin, wertvolle Proteinasen-Inhibitoren mit modifizierten Wirkungen und Wirksamkeiten im Vergleich zu Aprotinin sind (DE-OS 33 39 693; H. R. Wenzel et al 1985 in Chemistry of Peptides and Proteins, Band 3). Diese Aprotinin-Homologen weisen eine stark inhibierende Wirkung auf Elastasen aus Pankreas und Leukozyten auf.

Solche Aprotinin-Homologen können therapeutisch bei Erkrankungen in Verbindung mit einer übermäßigen Freisetzung von Pankreas-Elastase (Pankreatitis), Serum-Elastase (Arteriosklerose), Leukozyten-Elastase bei akuten und chronischen Entzündungen mit Schädigungen des Bindegewebes, bei Schäden an Gefäßwänden, nekrotischen Erkrankungen und der Degenerierung des Lungengewebes verwendet werden. Ebenso wichtig ist die Rolle, die lysosomale Enzyme, insbesondere Leukozyten Elastase, bei Entzündungsreaktionen aufgrund immunologischer Prozesse, z. B. rheumatoider Arthritis, spielen; außerdem können die genannten Homologen als Myocardunterdrückende Faktoren und bei Schocksyndromen verwendet werden.

Obwohl Aprotinin und Aprotinin-Homologe aus Rinderorganen und durch halbsynthetische Umwandlung von Rinder-Trypsininhibitor erhalten werden können (Tschesche, H., Wenzel, H., Schmuck R., Schnabel E. DE-OS 33 39 693 vom 15.5.1985),sind die Ausbeuten relativ gering.

Es wurde erkannt, daß die Anwendung rekombinanter DNA und assoziierter Technologien der erfolgreichste Weg wäre, um die erforderlichen großen Mengen an qualitativ hochstehenden Aprotinin-Homologen zur Verfügung zu stellen. Das Ziel war, Met-Aprotinin und Met-Aprotinin-Homologe, die biologisch aktiv sind, als nicht-fusionierte Produkte mit Hilfe der rekombinaten DNA-Technologie in einem Wirtsorganismus zu produzieren.

DNA, die für Polypeptide bekannter Aminosäuresequenz kodiert, kann hergestellt werden unter Verwendung der genomischen DNA-Sequenz, der cDNA-Sequenz, die komplementär zur mRNA ist, oder durch die Wahl von Codons gemäß dem genetischen Code und der Herstellung eines synthetischen Gens, die die entsprechenden Codons enthält

Eine DNA-Teilsequenz eines Rindergenom-Klons von Rinderpankreas-Trypsininhibitor-Gen wurde von S. Anderson und I.B. Kingston, 1983, Proc. Natl. Acad. Sci. USA, 80, 6838 - 6842 geklont, um einen genomischen Klon für Aprotinin zu charakterisieren. Kürzlich wurden ca. 4 kBP Segmente des Rindergenoms, der einen Kodierungsabschnitt für Rinderpankreas-Trypsininhibitor-Gen (BPTI e Aprotinin) und für Rindermilz-Inhibitor II enthält, von Kingston, I.B. und Anderson, S. 1986, Biochem. J. 233, 443-450, veröffentlicht.

Es konnte kürzlich gezeigt werden, daß Aprotinin-Homologe durch rekombinante DNA-Technologie unter Verwendung eines Konstrukts, bei welchem das Gen an ß-Galactase gebunden war, hergestellt werden können.

Es ist normalerweise viel leichter, die Expression eines Säugerproteins in einem bakteriellen Wirt mittels der Methode der Gen-Fusion durchzuführen, wobei man die Information für ein endogenes (homologes) Polypeptid, das natürlicherweise in einem bestimmten Genus oder einer Spezies der Wirtszellen existiert, mit der Information eines heterologen Polypeptids kombiniert, als daß man in einer Bakterien-Wirts-Zelle ein nicht-fusioniertes heterologes Polypeptid exprimiert. Das geht auf eine Reihe komplexer Phänomene zurück, die nicht vollständig bekannt sind. Einige der Faktoren, von denen man annimmt, daß sie die Expression oder Akkumulierung eines heterologen Polypeptids in einer Wirtszelle behindern, sind folgende:

1. Das heterologe Polypeptid kann durch die Wirtszelle abgebaut werden.
2. Das heterologe Polypeptid kann auf die Zelle einen toxischen Effekt ausüben.

Es wird angenommen, daß das Problem des Abbaus bei kleinen heterologen Polypeptiden - schwerwiegender ist als bei großen heterologen Polypeptiden.

Obwohl eine Vielzahl heterologer Polypeptide mit mehr als ca. 100 Aminosäureresten in E. coli exprimiert worden ist, hat man - trotz der zahlreichen Versuche - relativ wenige kleine heterologe Polypeptide in E. coli exprimiert.

Bei zahlreichen Versuchen, die Probleme der Exprimierung heterologer Polypeptide in Bakterienzellen zu überwinden, hat man sich der Fusionspolypeptide bedient.

Die Nachteile dieser Verfahrensweisen sind folgende:

1. Die insertierte Struktursequenz muß in bezug zum AUG-Startcodon des E. coli Gens im richtigen Leserahmen vorliegen.

2. Das heterologe Polypeptid muß aus dem Fusionspolypeptid mit Chemikalien herausgespalten werden, welche bestimmte Aminosäurereste modifizieren und/oder zerstören (z. B. Methionin durch Cyanogenbromid; Tryptophan durch Succinimid); deshalb ist es erforderlich, daß das heterologe Polypeptid frei von solchen Aminosäureresten ist.

Die Kopienzahl des zu exprimierenden Gens beträgt normalerweise 1 für jeden Expressionsvektor. Theoretisch ist es möglich, die Expression mehrerer Gene mit einem Genexpressionskontrollsystem zu regulieren (N. Lee et al 1984, Nucl. Acids Res. 12, 6797-6812).

Eine Erhöhung des Expressionsniveaus heterologer Polypeptide oder eine Stabilisierung eines exprimierten Polypeptids kann mit Hilfe von Amplifikationen von Domänen erzielt werden, wie dies von S-H Shen, 1984, Proc. Nat. Acad. Sci. USA 81, 4627-4631 gezeigt wurde. Er demonstrierte, daß mehrfache Kopien der für Proinsulin kodierenden Sequenz die Stabilität exprimierter Produkte, mit fusionierten und nicht-fusionierten Expressionssystemen, erhöhen konnte. Sein Produkt stellte ein vervielfachtes Polypeptid dar, das mit Cyanogenbromid gespalten werden muß. Das beste Expressionsniveau wurde mil drei Kopien erzielt.

Die Mechanismen, nach denen abnormale Proteine, einschließlich heterologen Polypeptiden, in E. coli abgebaut werden, sind nicht völlig bekannt. Durch Auswahl gewisser Wirtstämme können Polypeptide stabil produziert werden, die sonst instabil sind (Gottesmann, S. und Zipser, D., 1970 J. Bacteriol. 133, 844-851). Diese Mutanten stabilisieren jedoch nicht jedes heterologe in E. coli exprimierte Polypeptid.

Ein Gegenstand der Erfindung besteht in der zur Verfügungstellung von Met-Aprotinin, Met-Aprotinin-Homologen, Nukleinsäuren, die für diese kodieren, Vektoren, in welche die Nukleinsäuren inkorporiert sind, neue modifizierte Zellen, die damit transformiert sind und Verfahren zum Erhalt von Met-Aprotinin und Met-Aprotinin-Homologen. Die Bezeichnung Met-Aprotinin bezieht sich auf ein Aprotinin, in welchem die Aminosäure in Null(0)-Stellung ein Methionin darstellt.

Für die Genkonstruktion zur Herstellung synthetischer Gene war es besonders vorteilhaft, einen geeigneten Bauplan mit solchen Codons auszuwählen, die eine breite Anwendung ermöglichten. Dies ist besonders bei der Konstruktion eines synthetischen Mastergens der Fall, welches DNA-Blöcke oder Kassetten umfaßt, die von einmal vorkommenden Erkennungsstellen der Restriktionsenzyme terminiert werden. Eine solche Bauweise des Gens erlaubt eine leichte Modifizierung oder Mutation sämtlicher DNA-Sequenzen innerhalb solcher DNA-Blöcke, und wird von der vorliegenden Erfindung mitumfaßt.

Homologe von Aprotinin, die am Aminoende mit einem zusätzlichen Methionin versehen sind, wurden mit Hilfe der rekombinanten DNA-Technologie hergestellt. Es wurde festgestellt, daß solche Aprotinin-Homologe, wie z. B. Val-15-, Ile-15-, Leu-15-, Phe-15-, Ala-15-, Arg-15-, Gly-15-, Ser-15-, Thr-15-, Trp-15-, Met-15-und Tyr-15-Aprotinin dem bekannten Aprotinin und dessen Homologen äquivalent sind.

Es wird eine pharmazeutische Zubereitung in Einheits-Dosis oder flüssiger Form beschrieben.

Die vorliegende Erfindung umfaßt außerdem einen neuen Expressionsvektor, der Regulationssequenzen, wie Promotoren, Operatoren, Ribosomenbindungsstellen, Sequenzen für das Ribosomen-Pooling, Transkriptionsterminatoren, sowie typische Plasmidfunktionen und Gene oder Oligomere von Genen, welche die bakterielle Wirtszelle veranlassen, nicht-fusionierte heterologe Polypeptide zu exprimieren, umfaßt.

In einer weiteren Ausführungsform stellt die Erfindung ein Verfahren zur Herstellung von Genprodukten zur Verfügung, welche Oligomere des Gens umfassen, die mit den DNA-Sequenzen der Ribosomenbindungsstelle verbunden sind und für einzelne (nicht-fusionierte) Polypeptide kodieren, indem sie einen bakteriellen Wirt dazu veranlassen, die oligomerisierten Gene zu exprimieren.

Entsprechend dieser Ausführungsform wird ein Verfahren zur Oligomerisierung einzelner Gene mit einem speziellen Konstruktionsvektor offenbart, und es wird ein bakterieller Wirt beschrieben, der genetisch modifiziert wurde.

Die vorliegende Erfindung betrifft mikrobiell hergestelltes Met-Aprotinin und Met-Aprotinin-Homologe.

Das mikrobiell hergestellte Met-Aprotinin kann in Position 15 durch eine beliebige, natürlich vorkommende Aminosäure substituiert sein, insbesondere mit Arg, Val, Thr, Ile, Leu, Phe, Gly, Ser, Met, Trp, Tyr und Ala.

Das mikrobiell hergestellte Met-Aprotinin kann auch zusätzlich oder allein in Position 52 durch Glu, Leu, Val, Thr oder Ser substituiert sein. Auf diese Weise kann man mikrobiell hergestelltes Glu-52-Met-Aprotinin oder Val-15-Glu-52-Met-Aprotinin oder Ile-15-Glu-52-Met-Aprotinin oder Leu-15-Glu-52-Met-Aprotinin erhalten.

Die DNA, die für Met-Aprotinin kodiert, kann am Codon 15 und/oder am Codon 52 durch ein Codon substituiert sein, das für eine beliebige, natürlich vorkommende Aminosäure kodiert. Die DNA kann am Codon 15 durch ein Codon substituiert sein, das für eine Aminosäure kodiert, ausgewählt aus der Gruppe Arg, Val, Thr, Ile, Leu, Phe, Gly, Ser, Trp, Tyr, Met und Ala. Am Codon 15 kann die DNA durch ein Codon substituiert sein, das für eine Aminosäure kodiert, die ausgewählt ist aus der Gruppe Glu, Leu, Val, Thr und Ser.

Die Erfindung betrifft im weiteren eine DNA, die für ein Protein oder ein Polypeptid kodiert, und welche in 5'Richtung die DNA der folgenden Sequenz

```
                RBS
CTA GAT TAAAGGAGTTATCTT
        TA ATTTCCTCAATAGAA
```

oder funktionelle Äquivalente derselben aufweist sowie auch eine DNA, die für ein Protein oder ein Polypeptid kodiert, und welche in 3'Richtung mit einer DNA der folgenden Sequenz

```
GCA
CGTTCGA
```

oder funktionellen Äquivalenten derselben ausgestattet ist.

Die Erfindung betrifft insbesondere eine DNA, die für ein Protein oder Polypeptid kodiert, welche in 5'Richtung die DNA der folgenden Sequenz

```
                RBS
CTA GAT TAAAGGAGTTATCTT
        TA ATTTCCTCAATAGAA
```

und in 3'Richtung die DNA der folgenden Sequenz

```
GCA
CGTTCGA
```

oder funktionelle Äquivalente derselben aufweist.

Der kodierende Teil der DNA kann z. B. für folgende Polypeptide kodieren: Insulin, Proinsulin, Vasopressin, Oxytocin, Ribonuklease, Wachstumshormon, Met-Aprotinin und Homologe von Met-Aprotinin.

Im weiteren betrifft die vorliegende Erfindung ein Expressionssystem für ein Protein oder für ein Polypeptid, welches eine Kopie oder mehrere Kopien einer DNA umfaßt, die für ein Protein oder ein Polypeptid kodiert, wobei jede Kopie der kodierenden DNA in 5'Richtung eine DNA der folgenden Sequenz

```
                  RBS
CTA GAT TAAAGGAGTTATCTT
        TA ATTTCCTCAATAGAA
```

und in 3'Richtung eine DNA der folgenden Sequenz

```
GCA
CGTTCGA
```

und funktionelle Äquivalente dieser Sequenzen aufweist.

Eine DNA der Sequenz

```
AGCTAATGAGCGCGC
    TTACTCGCGCGATC
```

wird beschrieben. Diese DNA ist eine DNA-Sequenz mit einer Funktion als Adaptor.

Außerdem wird eine DNA der Sequenz

```
                              RBS
GCAAGCTAATGAGCGCGCCTAGATTAAAGGAGTTATCTT
CGTTCGATTACTCGCGCGGATCTAATTTCCTCAATAGAA
             BssHII
```

beschrieben. Diese DNA stellt eine DNA-Sequenz mit einer Funktion als Linkersequenz dar.

Im weiteren umfaßt die Erfindung eine DNA mit der folgenden Sequenz:

RBS

```
AAGGGATATCTT ATG ACA AGC TTT CTA GAT
TTCCCTATAGAA TAC TGT TCG AAA GAT CTA
```

EcoRV HindIII XbaI

Diese DNA kodiert für ein kurzes Peptid und hat eine Funktion als Expressionsverstärker. Es wird eine DNA beschrieben mit einer Funktion als Operator mit folgender Sequenz

mRNA lac Operator RBS

```
ATCTAGCAAATTGTGAGCGGATAACAATTTGCACACAGCTAGATAATAAAAATTTAAGGGAT
TAGATCGTTTAACACTCGCCTATTGTTAAACGTGTGTCGATCTATTATTTTTAAATTCCCTA
```

EcoRV

und eine DNA mit einer Funktion als Promotor mit folgender Sequenz:

-35 Promotor -10

```
CTCGAGATAAAAAATTTATTTGCTTTCAGGTACAATTCTTGATATAATATTATC
GAGCTCTATTTTTTAAATAAACGAAAGTCCATGTTAAGTTCATATTATAATAG
```

XhoI

Dem Fachmann auf dem entsprechenden Gebiet ist es bekannt, daß die Substitution einer oder mehrerer Basen in der beschriebenen Sequenz nicht notwendigerweise zu einem Verlust oder einer Änderung der Funktion führt. Deshalb umfaßt die Erfindung auch funktionelle Äquivalente der genannten Sequenzen.

Beide DNA-Sequenzen können zu einer DNA kombiniert werden, die eine Funktion als Promotor und Operator hat und die folgende Sequenz aufweist

-35 Promotor -10

```
CTCGAGATAAAAAATTTATTTGCTTTCAGGTACAATTCTTGATATAATATTATC
GAGCTCTATTTTTTAAATAAACGAAAGTCCATGTTAAGTTCATATTATAATAG
```

XhoI

mRNA lac Operator RBS

```
ATCTAGCAAATTGTGAGCGGATAACAATTTGCACACAGCTAGATAATAAAAATTTAAGGGAT
TAGATCGTTTAACACTCGCCTATTGTTAAACGTGTGTCGATCTATTATTTTTAAATTCCCTA
```

EcoRV

oder die funktionelle Äquivalente der genannten Sequenz aufweist.

Das vorstehend beschriebene Expressionssystem kann in 5'Richtung mit den DNA-Sequenzen versehen sein, die sich aus der DNA mit einer Funktion als Promotor und Operator zusammensetzt und dann in ein Plasmid inkorporiert werden.

Die Bezeichnung "Expressionssystem" bezieht sich auf eine DNA-Sequenz, welche eine Struktursequenz, die für ein Protein oder Polypeptid kodiert, und regulatorische Sequenzen, wie Promotor-und Operator, umfaßt.

Dieses Plasmid eignet sich zum Transformieren eines Mikroorganismus, insbesondere eines E. coli Mikroorganismus.

Am meisten bevorzugt ist ein E. coli BR17 Mikroorganismus, der mit dem Plasmid transformiert wird und die DSM-Bezeichnung DSM 3685 und DSM 3686 trägt.

Die Erfindung betrifft Plasmide, die die Restriktions-Karte von PiWiT 10 wL1 und PiWiT 11 aufweisen sowie auch die Plasmide piWiT10 wi7 und piWi T11.

Die Plasmide piWiT 10 wL1 und piWiT 11 eignen sich in einem Verfahren zur Herstellung von Proteinen und Polypeptiden, insbesondere einem Verfahren zur Herstellung eines Polypeptids ausgewählt aus der Gruppe Insulin, Proinsulin, Vasopressin, Oxytocin, Ribonuklease, Wachstumshormon, Met-Aprotinin und Homologe von Met-Aprotinin.

Die gemäß der Erfindung verwendeten Mikroorganismen wurden bei der deutschen Sammlung von Mikroorganismen, Griesbachstrasse 8, D-3400 Göttingen, hinterlegt. Die DSM-Hinterlegungsnummer des Wirt-Mikroorganismus E. coli BR 17 ist DSM 3684.

Die DSM-Hinterlegungsnummer des Wirt-Mikroorganismus E. Coli BR 17, der mit den Plasmiden piWiT10 wi11 und piWiT10 wi7 transformiert ist, ist DSM 3685 und DSM 3686.

Kurze Beschreibung der Zeichnungen

Fig. 1a. Aufbau des synthetischen Gens
Fig. 1b. DNA-Sequenz von DNA-Fragmenten
Fig. 2a. Konstruktionsvektor piWi T9
Fig. 2b. DNA-Teilsequenzen von piWi T9
Fig. 3. Amplifikationsschema zur Oligomerisation des synthetischen Gens
Fig. 4a. Expressionsvektor piWi T 11
Fig. 4b. DNA-Teilsequenz von piWi T11
Fig. 5a. Plasmid PiWi T10 wL1
Fig. 5b. DNA-Teilsequenzen von piWi T10 wL1
Fig. 6. SDS-Protein-Gelelektrophorese
Fig. 7. SDS-Protein-Gelelektrophorese
Fig. 8. Renaturierung von Met-Aprotinin
Fig. 9. Western-Blot
Fig. 10. Chromatographie von Met-Aprotinin

Im folgenden werden die Strategie für die Konstruktion und Selektion, die Herstellung von DNA-Fragmenten, die für Met-Aprotinin, Met-Aprotinin-Homologe kodieren, Konstruktionsvektoren, Expressionsvektoren, Expressionsplasmide, Mikroorganismen, die mit denselben transformiert sind, und die mikrobielle Herstellung von Met-Aprotinin und Met-Aprotinin-Homologen, sowie pharmazeutische Zubereitungen offenbart.

Es wurden Standardmethoden der rekombinanten DNA-Technik angewendet, wie sie von Maniatis et al, 1982, Molecular Cloning, Cold Spring Harbor Laboratory, Cold Spring Harbor, USA beschrieben sind, wobei manchmal Modifikationen angewendet werden, wie sie im folgenden beschrieben werden.

<u>Strategie der Konstruktion und Selektion von DNA-Fragmenten, die ein synthetisches Inhibitorgen kodieren</u>

Die bekannte Proteinsequenz und der genetische Code von Aprotinin und Aprotinin-Homologen dienten zur Bestimmung einer DNA-Sequenz, die für solche Polypeptide kodiert.

Die Degenerierung des genetischen Codes erlaubt eine bestimmte Freiheit in der Wahl der Codons für eine gegebene Aminosäuresequenz.

Alle möglichen Basensubstitutionen unter den Codons, die die Aminosäuresequenz dieses Proteins festlegen, wurden bestimmt. Dementsprechend wurden alle potentiellen Restriktionsstellen, die innerhalb der möglichen DNA-Sequenzen lokalisiert sind, bestimmt.

Die Codonwahl für Mastergene war von folgenden Überlegungen begleitet:

1. Es wurden Codons und Fragmente ausgewählt und die Fragmentzusammensetzung geplant, und zwar so, daß eine nicht gewünschte Komplementarität der Fragmente vermieden wurde.

2. Der Bereich oberhalb des Initiationscodons enthält eine ribosomale Bindungsstelle: als Codons um den Bereich des Initiationscodons wurden solche ausgewählt, die die Ribosomenbindung verstärken (Lit. G.F.E. Scherer et al. 1980, Nucl. Acids Res. 8, 3895-3907).

3. Es wurden Restriktionsstellen ausgewählt, die erforderlich sind zur Erleichterung der Bestimmung von Transformanden oder zum Austausch von Basen mittels Ersatz geeigneter Fragmente durch andere Fragmente, so daß man leicht Modifikationen von Aprotinin herstellen kann, sowie zur Schaffung eines breiten Spektrums an Möglichkeiten zur Klonierung und Expression.

4. Die Mehrheit der gewählten Codons sind solche, die bei der Expression mikrobieller Gene bevorzugt vorkommen (siehe H. Grosjean and W. Fiers. Gene, 18 (1982) 192-209: M. Gouy und C. Gautier, Nucleic Acids Research, 10 (1982) 7055 - 7074).

Der prinzipielle Bauplan für synthetische Aprotiningene und deren Homologe ist in Fig. 1a. wiedergegeben.

Konstruktion synthetischer Gene

Die synthetischen Gene für Met-Aprotinin-Homologe wurden über ein Mastergen durch Zusammensetzung von 14 gereinigten Oligonukleotiden konstruiert. Diese Konstruktion erfolgte wie in Fig. 1a. gezeigt. Die DNA-Sequenzen der Fragmente für den synthetischen Inhibitor gehen aus Fig. 1b. hervor.

Das Mastergen umfaßt eine Ribosomenbindungsstelle, das Initiationscodon ATG, das Terminationscodon, TAG, die 5'-terminale Restriktionsstelle für Xba I, die 3'-terminale Restriktionsstelle für Hind III, zusätzlich die internen Restriktionsstellen für Xho I, Apa I, Stu I, Acc I. Pst I. Sst II und Sph I. Diese Restriktionsstellen, insbesondere die internen, erleichtern das Klonieren der kodierenden Sequenz, die Modifikation des Mastergens durch Austausch von DNA-Fragmenten, die für andere Aminosäuren kodieren oder die einen anderen Codongebrauch haben. Das gesamte Spektrum des Proteinengineerings ist mit einem solchen Konstrukt möglich.

Um Gene für Met-Aprotinin-Homologe zu konstruieren, ist lediglich der Austausch eines Restriktionsfragmentes mit einer geeigneten DNA-Sequenz erforderlich. Sequenzen für solche Fragmente, die für Aminosäureänderungen in Position 15 kodieren, z. B. Apa I - Stu I-Fragmente, werden in Fig. 1b. wiedergegeben.

Konstruktionsvektor piWi T9 und Amplifikation der Gene

Das für experimentelle Met-Aprotinin-Klonierung gewählte Plasmid war piWi T9 (siehe auch Fig. 2a.).

Der Konstruktionsvektor setzt sich zusammen aus zwei Fragmenten von pBR 322, welche jeweils den Ursprung der Replikation ("origin of replication") und das Tetracycinresistenzgen tragen, einem Fragment der Bakteriophagen-fd 11-DNA, welches ein Transkriptions-Terminationssignal trägt, und einem Fragment aus dem lac-Operon von E. coli, welches sich erstreckt vom C-Terminus des lac-I-Gens bis genau hinter das lac-Z-gen, wobei die gesamte lac-Promotor-Operator-Region und die ersten fünf Codons des lac-Z-Gens deletiert und durch ein kurzes Stück einer synthetischen DNA ersetzt wurden. Die synthetische DNA besitzt konstitutive Promotoraktivität, welche auf das lac-Z-Gen gerichtet ist und sie enthält die Klonierungsstellen Xba I, Xma I und Hind III zwischen dem Promotor und dem lac-Z-Gen. Jede DNA, die in eine beliebige dieser drei Stellen kloniert ist, wird von dem synthetischen Promotor transkribiert: ob sie auch translatiert wird hängt von der Sequenz ab. Das lac-Z-Gen von piWi T9 wird nicht translatiert, es wird nur dann aktive ß-Galactosidase hergestellt, wenn zusätzliche DNA in eine beiliebige der Klonierungsstellen eingefügt wird, die ein Initiationscodon im Leseraster für das lac-Z-Gen mitbringt.
Die DNA-Sequenzen wichtiger Bereiche des Konstruktionsvektors piWi T9 sind in Fig. 2b aufgeführt.

Zur Oligomerisation von Genen wurde das synthetische Gen (siehe Fig. 1a) zwischen die Restriktionsstellen Xba I und Hind III des Konstruktionsvektors piWi T9 kloniert. Für eine Tandemverdoppelung des synthetischen Gens wurde ein Bam HI - Hind III-Fragment, welches eine Kopie des oberhalb der Hind III-Stelle lokalisierten Gens enthält, in Gegenwart eines Hind III-Xba I-Adaptors an ein Xba I - Bam HI-Fragment, welches eine Kopie des unterhalb der Xba I-Stelle lokalisierten Gens enthält (s. Fig. 3) ligiert. Der Hind III - Xba I-Adaptor paßt in die überstehenden Enden, wie sie von Hind III und Xba I gebildet werden, regeneriert jedoch diese Stellen nicht. Das erhaltene Plasmid piWi T9 TL2 hat eine einzige Xba I-Stelle oberhalb des ersten Gens und eine einzige Hind III-Stelle unterhalb des zweiten Gens. Somit kann die Verdopplung mit Hilfe der gleichen Reihe von Reaktionen wiederholt werden, wobei man weitere Plasmide erhält, die 4. 8. 16 und 32 in Tandemanordnung wiederholte Gene enthalten, wobei jedem seine eigene ribosomale Bindungsstelle vorausgeht. Da das lac-Z-Gen nicht mehr gebraucht wird, wird das 3kb Eco RI-Fragment aus dem Plasmid deletiert.

Expressionsvektor piWi T11 und Expressionsplasmid piWi T10 wL1

Zur Expression von Met-Aprotinin und Met-Aprotinin-Homologen wurde ein Plasmid konstruiert, in welchem die geeigneten Gene unter Kontrolle eines starken Promotors-Operators exprimiert werden konnten, und welches im weiteren DNA-Sequenzen umfaßt, die zur Expression heterologer Proteine und Polypeptide sehr geeignet sind. Die physikalische Karte des Expressionsvektors piWi T11 ist in Fig. 4a wiedergegeben.

Der Expressionsvektor piWi T11 ist dem Konstruktionsvektor piWi T9 sehr ähnlich; sie besitzen ein gemeinsames 3,3 Kb-langes Xho I - Eco RI-Fragment, welches 36 bp lac I-DNA, 883 bp pBR 322 DNA (Ursprung der Replikation), 2067 (+ 8) bp pBR 322 DNA (Tetracyclinresistenzgen), 9 bp Polylinker, 332 bp fd 11 DNA-Transkriptions-Terminationssignal, 8 bp Bam HI-Octalinker und 68 bp lac-Z-DNA umfaßt. Um die Expression zu verstärken, wurde ein synthetisches DNA-Fragment konstruiert und zwischen die Eco RI - Xho I-Stellen ligiert. Die Sequenz dieses synthetischen Fragments ist in Fig. 4b wiedergegeben. Das Fragment umfaßt Sequenzen für einen starken Promotor, einen RNA-Start, eine lac-Repressor-Bindungsstelle (Operator), eine ribosomale Bindungsstelle, ein Protein-Startcodon, einen kurzen kodierenden Abschnitt, der fünf einmal vorkommende Restriktionsstellen zur Klonierung enthält (Hind III, Xba I, Pst I, Bgl II and Xma I).

Für Expressionszwecke wurde das kleine Xho I - Xba I-Fragment eines Konstruktionsvektors mit Oligomeren des synthetischen Gens, wie oben beschrieben, durch ein kleines Xho I - Xba I-Fragment von piWi T11 ausgetauscht, wobei letzteres einen Promotor, Operator, eine Ribosomenbindungsstelle und eine Kodierungssequenz für ein Hexapeptid enthielt. Ein erhaltenes rekombinantes Plasmid ist z. B. das Expressionsplasmid piWi T10 wL1 mit Oligomeren des Met-Aprotiningens.

DNA-Sequenzen wichtiger Abschnitte des Expressionsplasmids sind in Fig. 5b wiedergegeben.

Der Mikroorganismus

Im Stand der Technik kennt man eine große Zahl an Mikroorganismen, die sich zur Transformation eignen. Das sind solche einzelligen Organismen, die zur Züchtung in Kulturen oder für Fermentationen geeignet sind. Bevorzugte Organismen zur Transformation umfassen Bakterien, Hefen und Pilze.

Der für die vorliegende Arbeit gewählte Organismus war E. coli.

Ein besonders geeigneter Stamm ist E. coli B lamda $R^-$, $mal^-$, der mit Hilfe der von J.H. Miller, 1972, Experiments in Molecular Genetics, Cold Spring Harbor Laboratory, beschriebenen Methode zu recA notiert wurde.

Herstellung von Met-Aprotinin

Viele Proteine, die in großen Mengen in Bakterien synthetisiert werden, akkumulieren sich in unlöslicher Form (D. C. Williams, R.M. van Frank, J.B. Burnett, W.L. Muth, 1982, Science 215, 687).

Diese unlöslichen Proteine bezeichnet man als Inklusionskörper. Sie können gewöhnlich nur mit polaren Stoffen, wie z. B. Harnstoff oder Guanidiniumhydrochlorid gelöst werden.

Der E. coli Stamm BR17 wurde mit dem Plasmid piWi T10 wL1 transformiert, welches für das Aprotiningen vierzehnmal unterhalb eines E. coli Promotors, Operators und einer Ribosomen-Bindungsstelle kodiert. Eine Übernacht-Kultur von E. coli Stamm BR17 piWi T10 wL1 wurde zentrifugiert, das Pellet wurde in Puffer zum Aufbrechen resuspendiert, und die Zellen wurden mittels einer "french press". lysiert.

Das Zell-Lysat wurde zur Gewinnung der Inklusionskörper zentrifugiert. Das Pellet wurde zweimal mit Puffer (breaking puffer) gewaschen.

Die Reinigungsschritte wurden mittels der SDS-Polyacrylamid-Gelelektrophorese nach Laemmli (U.K. Laemmli 1970. Nature 277. 680-685) überprüft; Fig. 7.

Der inaktive Inhibitor wurde nach einem Verfahren von Creighton (T.E. Creighton. Proceedings of Genex-UCLA Symposium 1985 Kingstones) renaturiert; Fig 8. Der aktive Inhibitor konnte durch Trypsinbestimmung und Western-Blot-Analyse nachgewiesen werden; Fig. 9.

Der aktive Inhibitor wurde im weiteren durch Chromatographie auf einer Trypsin-Sepharose-Säule nach Fritz (H. Fritz. M. Gebhardt. R. Meister. K. Illchmann, K. Hochstraßer. 1970. Hoppe-Seyler's Z. Physiol. Chem. 351. 571-574) gereinigt; Fig. 10.

Der Inhibitor wurde dann durch Mikrosequenzierung gemäß Hewick (R.M. Hewick. M.W. Hunkapillar. L.E. Hood. W.I. Dreger 1981. J. Biol. Chem. 256. 7970-7997) charakterisiert.

Die ersten 20 Reste am N-Terminus wurden bestimmt. Abgesehen von dem Methionin in der Position Null war die Aminosäuresequenz völlig mit der von Aprotinin identisch (Tabelle 1). Die Aminosäurezusammensetzung des Inhibitors zeigt die erwarteten Werte (Tabelle 2). Ein Vergleich von Aprotinin und Met-Aprotinin mittels der Trypsin-inhibierenden Wirkung zeigt vergleichbare Werte (Tabelle 3).

Alle diese Experimente zeigen, daß es möglich ist, Met-Aprotinin in E. coli herzustellen und es zum aktiven Inhibitor zu renaturieren.

Herstellung von Met-Ile-15-Aprotinin

Met-Ile-15-Aprotinin kann in ähnlicher Weise in E. coli hergestellt werden, wie dies für Met-Aprotinin beschrieben ist (siehe auch Fig. 6).

Die inhibierende Wirkung wurde mittels eines Elastase-Inhibierungsassays (K. Nakajima, M. Zimmermann, J.C. Powers, M.J. Castillo, B.M. Ashe 1979, J. Biol. Chem. 254, 4027) bestimmt.

Der Inhibitor wurde durch Aminosäureanalyse und N-terminale Sequenzierung charakterisiert (Tabelle 1, 2).

Alle anderen Derivate von Aprotinin konnten in ähnlicher Weise hergestellt werden, wie dies für Met-Aprotinin und Met-Ile-15-Aprotinin beschrieben ist.

Pharmazeutische Zubereitungen

Die vorliegende Erfindung umfaßt pharmazeutische Zubereitungen, die außer nicht-toxischen, inerten, pharmazeutisch geeigneten Exzipienten eine oder mehrere Verbindungen gemäß der Erfindung umfassen, oder die aus einer oder mehreren aktiven Verbindungen gemäß der Erfindung bestehen, sowie Verfahren zur Herstellung dieser Zubereitungen.

Die vorliegende Erfindung umfaßt auch pharmazeutische Zubereitungen in Dosis-Einheiten. Dies bedeutet, daß die Zubereitungen in Form individueller Teile vorliegen, z. B. als Tabletten, beschichtete Tabletten, Kapseln, Pillen, Suppositorien und Ampullen, wobei der Gehalt an aktiver Verbindung einem Teil oder einem Vielfachen einer individuellen Dosis entspricht. Die Dosiseinheiten können z. B. eine, zwei, drei oder vier individuelle Dosen oder eine Hälfte, ein Drittel oder ein Viertel einer individuellen Dosis enthalten. Eine individuelle Dosis enthält bevorzugt die Menge an aktiver Verbindung, die bei einer Verabreichung gegeben wird, und die üblicherweise dem Ganzen, einer Hälfte, einem Drittel oder einem Viertel einer Tagesdosis entspricht.

Unter nicht-toxischen, inerten, pharmazeutisch geeigneten Exzipienten versteht man feste, halbfeste oder flüssige Verdünner, Füllmittel und Formulierungshilfsmittel aller Art.

Als bevorzugte pharmazeutische Zubereitungen sind Tabletten, beschichtete Tabletten, Kapseln, Pillen, Granalien, Suppositorien, Lösungen, Suspensionen und Emulsionen, Pasten, Salben, Gele, Cremen, Lotionen, Pulver und Sprays zu nennen.

Tabletten, beschichtete Tabletten, Kapseln, Pillen und Granalien können die aktive Verbindung oder die aktiven Verbindungen zusammen mit üblichen Exzipienten enthalten, wie z. B. (a) Füllmittel und Streckmittel, z. B. Stärken, Lactose, Sucrose, Glukose, Mannit und Siliziumdioxid, (b) Bindemittel, z. B. Carboxymethylcellulose, Alginate, Gelatine und Polyvinylpyrrolidon, (c) anfeuchtende Mittel, z. B. Glycerin, (d) Disintegrationsmittel, z. B. Agar-Agar, Kalziumcarbonat und Natriumcarbonat, (e) Lösungsverzögerer, z. B. quaternäre Ammoniumverbindungen, (g) Benetzungsmittel, z. B. Cetylalkohol und Glycerinmonostearat, (h) Adsorbentien, z. B. Kaolin und Bentonit, und (i) Schmiermittel, z. B. Talk, Kalzium-und Magnesiumstearat und feste Polyethylenglykole, oder Gemische der unter (a) bis (i) aufgeführten Substanzen.

Die Tabletten, beschichtete Tabletten, Kapseln, Pillen und Granalien können mit üblichen Beschichtungen und Umhüllungen versehen werden, und enthalten gegebenenfalls Opazifizierungsmittel; sie können auch eine Zubereitung darstellen, welche die aktive Verbindung oder die aktiven Verbindungen nur oder vorzugsweise in einem bestimmten Teil des Intestinaltraktes, gegebenenfalls in verzögerter Weise, freisetzen. Beispiele von geeigneten Einbettungsmaterialien sind polymere Substanzen und Wachse.

Die aktive Verbindung oder Verbindungen, gegebenenfalls zusammen mit einem oder mehreren der vorstehend genannten Exzipienten, kann auch in einer mikroverkapselten Form vorliegen.

Suppositorien können außer der aktiven Verbindung oder den aktiven Verbindungen die üblichen wasserlöslichen oder wasserunlöslichen Exzipienten aufweisen, z. B. Polyethylenglykole,Fette, z. B. Kakaofett und höhere Ester (z. B. $C_{14}$-Alkohol mit $C_{16}$-Fettsäure) oder Gemische dieser Substanzen.

Salben, Pasten, Cremen und Gele können übliche Exzipienten zusätzlich zu der aktiven Verbindung oder den aktiven Verbindungen enthalten, z. B. tierische und pflanzliche Fette, Wachse, Paraffine, Stärke, Tragacanth, Cellulosederivate, Polyethylenglykole, Silikone, Bentonite, Siliziumdioxid, Talk und Zinkoxid, oder Gemische dieser Substanzen.

Pulver und Sprays können übliche Exzipienten neben der aktiven Verbindung oder den aktiven Verbindungen enthalten, z. B. Lactose, Talk, Siliziumdioxid, Aluminiumhydroxid, Kalziumsilikat und Polyamidpulver, oder Gemische dieser Substanzen. Sprays können außerdem übliche Treibmittel enthalten, z. B. Chlorfluorkohlenwasserstoffe.

Lösungen und Emulsionen können übliche Exzipienten neben der aktiven Verbindung oder den aktiven Verbindungen enthalten, wie Lösungsmittel, Solubilisierungsmittel und Emulgiermittel, z. B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsamenöl, Erdnußöl, Maiskeimöl, Olivenöl, Rizinusöl und Sesamöl, Glycerin, Glycerinformal, Tetrahydrofurfurylalkohol, Polyethylenglykole und Fettsäureester von Sorbitan, oder Gemische dieser Substanzen.

Zur parenteralen Verabreichung können die Lösungen und Emulsionen auch in einer sterilen Form, die isoton mit Blut ist, vorliegen.

Die Suspensionen können übliche Exzipienten neben der aktiven Verbindung oder den aktiven Verbindungen enthalten, wie flüssige Verdünnungsmittel, z. B. Wasser, Ethylalkohol oder Propylenglykol, Suspensionsmittel, z. B. ethoxylierte Isostearylalkohole, Polyoxyethylensorbit und Sorbitanester, mikrokristalline Cellulose, Aluminium-metahydroxid, Bentonit, Agar-Agar und Tragacanth, oder Gemische dieser Substanzen.

Die genannten Formulierungsformen können auch Farbstoffe, Konservierungsmittel und Additive enthalten, die den Geruch und Geschmack verbessern, z. B. Pfefferminzöl und Eukalyptusöl, und Süßungsmittel, z. B. Saccharin.

Die therapeutisch wirksamen Verbindungen sollten in den vorstehend genannten pharmazeutischen Zubereitungen bevorzugt in einer Konzentration von ca. 0,1 bis 99,5, vorzugsweise von 0,5 bis 95 Gew.-%, bezogen auf das Gesamtgemisch, vorliegen.

Die vorstehend genannten pharmazeutischen Zubereitungen können auch andere pharmazeutisch aktive Verbindungen zusätzlich zu den Verbindungen gemäß der Erfindung enthalten.

Die vorstehend genannten pharmazeutischen Zubereitungen werden in üblicher Weise nach bekannten Verfahren hergestellt, z. B. durch Vermischen der aktiven Verbindung oder der aktiven Verbindungen mit dem oder den Exzipienten.

Die aktiven Verbindungen oder die pharmazeutischen Zubereitungen können lokal, oral, parenteral, intraperitoneal und/oder rektal verabreicht werden, bevorzugt oral oder parenteral, wie intravenös oder intramuskular.

Im allgemeinen hat es sich sowohl in der Human-als auch in der Veterinärmedizin als vorteilhaft erwiesen, die aktive Verbindung oder die aktiven Verbindungen gemäß der Erfindung in Gesamtmengen von ca. 0,5 bis ca. 500, vorzugsweise 5 bis 100 mg/kg Körpergewicht alle 24 h, gegebenenfalls in Form mehrerer individueller Verabreichungen, zu geben, um die gewünschten Ergebnisse zu erzielen. Eine individuelle Verabreichung enthält die aktive Verbindung oder die aktiven Verbindungen gemäß der Erfindung vorzugsweise in Mengen von ca. 1 bis ca. 250, insbesondere von 3 bis 60 mg/kg Körpergewicht. Es kann jedoch erforderlich sein, von dieser genannten Dosis abzuweichen, insbesondere in Abhängigkeit von der Natur und dem Körpergewicht des zu behandelnden Patienten, der Art und Schwere der Erkrankung, der Art der Zubereitung und der Verabreichung der Medizin, und der Zeit oder dem Intervall, über den die Verabreichung stattfindet.

Somit kann es in einigen Fällen genügen, mit weniger als der vorstehend genannten Menge an aktiver Verbindung auszukommen, während in anderen Fällen die vorstehend genannte Menge an aktiver Verbindung überschritten werden muß. Die insbesondere erforderliche optimale Dosis und die Art der Verabreichung der aktiven Verbindung kann ohne weiteres vom Fachmann auf der Grundlage seines Fachwissens bestimmt werden.

Beispiele

Beispiel 1

Synthese und Reinigung von DNA-Fragmenten, die für Met-Aprotinin und Met-Ile-15-Aprotinin kodieren

Die Oligonukleotide, die das Gen umfassen, wurden unter Anwendung von Festphasen-Synthesemethoden hergestellt. Das Syntheseschema für die Oligomeren entsprach dem angegeben und bediente sich Protonen-aktivierter, geschützter 2'-Desoxyribonukleotid-phosphoramidite. Sämtliche Sequenzschritte wurden in automatisierter Weise auf einem Applied Biosystems Modell 380 DNA-Synthesator unter Verwendung geschützter Nukleotide, Lösungsmittel, Chemikalien und Reagentien, die vom Hersteller bezogen wurden, ausgeführt. Der Festphasen-Träger, der vom gleichen Hersteller ist, war c"controlled pore glass" an welchem das Ausgangs-3'-nukleotid bereits aufgebracht war.. Bestimmte Modifikationen wurden in den automatisierten Reaktionszyklus in Übereinstimmung mit den "Manufacturers Operating Instructions" and "Users Bulletins" eingeführt. Nach Beendigung der Synthese wurden die Oligomeren entblockt und vom Festphasen-Träger innerhalb des DNA-Synthesators, wie vom Hersteller angegeben, abgespalten.

Die Entfernung der Blockierungsgruppen wurde durch Erwärmen der wäßrigen Lösung, die das Oligomer enthält, mit konzentriertem Ammoniumhydroxid für 4 bis 24 h auf 55°C in einer versiegelten Ampulle vervollständigt. Die erhaltene Lösung wurde eingedampft, der Rückstand in 0,01 M Triethylammonium-bicarbonat-Puffer, pH 7,0 (TEAB-Puffer) aufgelöst. Diese Lösung wurde über Sephadex-G 50 ® -Gelfiltrationsharz chromatographiert.

Die Säule wurde in dem gleichen TEAB-Puffer hergestellt und mit diesem wurde eluiert. Das Material, das mit dem Leervolumen eluierte, wurde vereinigt und die Lösung eingedampft.

Ein Teil des Rückstandes (10 bis 40 % der Extinktionseinheiten bei 260 nm), aufgelöst in Beladungspuffer (Zusammensetzung: 0,1 % Bromphenol-Blau, 0,1 % Xylolcyanol, 10 mM Dinatrium-EDTA, in Formamid) wurde durch Elektrophorese in Polyacrylamidgelen weiter gereinigt. Die Gelabmessung betrug 10 $\times$ 32 cm mit einer Dicke von 1,5 mm. Die Geltasche für jedes auf diese Weise gereinigte Oligomer war 2 bis 5 cm breit; bis zu fünf Oligomere wurden unter Verwendung eines Gels gereinigt. Die Acrylamid-Konzentration des jeweiligen Gels variierte von 14 bis 20 %, je nach der Kettenlänge zu reinigenden Produkte. Für längere Oligomere wurde das 14 %ige Acrylamidgel bevorzugt, während kürzere Oligomere auf bis zu 20 %igen Acrylamidgelen gereinigt wurden. Die Gele enthielten auch 7 M Harnstoff und Tris-borat-EDTA-Puffer (0,1 M Tris, 0,1 M Borat, 2 mM EDTA, pH 8,3). Der Laufpuffer bestand ebenfalls aus dem Tris-borat-EDTA-Gemisch. Die Elektrophorese wurde bei 20 bis 60 Watt, konstantem Strom, 6 bis 18 h lang durchgeführt. Derartige standardisierte Methoden sind verschiedenen "User Information Bulletins" von Applied Biosystems zu entnehmen.

Nach Beendigung der Elektrophorese wurde das Gel auf eine Plastikfolie gelegt und die Oligomeren durch "UV-shadowing" sichtbar gemacht. Diese Abbildung erreicht man, indem man das Gel mit Folie auf eine Fluoreszenz-Dünnschicht-Chromatographieplatte legt und das Gel unter einer kurzwelligen UV-Lichtquelle beobachtet.

Das gewünschte Produkt erscheint als oas am langsamsten wandernde, blaue DNA-Haupt-Fragment mittels dieser Abbildungs-Methode. Die gewünschte 8ande wird aus dem Gel herausgeschnitten. Das DNA-Oligomer wird aus der Gelscheibe, auf pulverförmiger Diethylaminoethyl(DEAE)-cellulose unter Verwendung einer EpiGene D-Gel®-Elektrophoreseapparatur eluiert. Das Oligomer wird aus der Cellulose durch Eluieren mit 1 M TEAB-Puffer gewonnen. Die Pufferlösung, die das Oligomer enthält, wird eingedampft, der Rückstand in 0,01 M TEAB-Puffer aufgelöst und dann durch Chromatographie über eine Sephadex-G 50®-Säule, wie vorstehend beschrieben, entsalzt. Das im Leervolumen eluierende Material wird vereinigt und unter Erhalt des Endproduktes lyophilisert.

Unter Anwendung der vorstehend beschriebenen Verfahren wurden ca. 0,5 bis 5,0 $A_{260}$-Einheiten eines jeden der gereinigten Oligomere erhalten.

Beispiel 2

Konstruktion von synthetischen Genen

Die synthetischen Oligonukleotide, die wie in Beispiel 1 gereinigt worden waren, wurden mit Polynukleotidkinase und Adenosintriphosphat (ATP) unter Standardbedingungen behandelt (Maniatis et al.(1982)). Ein Zehntel einer jeden Probe wurde mit δ[32P] ATP anstelle von ATP inkubiert. ßeide Proben wurden anschließend vermischt und, wie in Beispiel 1 beschrieben. einer Elektrophorese unterworfen. Die markierten Banden wurden aus dem Gel eluiert und wie zuvor beschrieben gereinigt.

Die 14 Oligonukleotide (Fig. 1a, b) wurden dann mit 30 µl doppelt-konzentriertem Ligase-Puffer (Maniatis et al. (1982 vermischt und 20 min bei 40°C und 30 min bei Raumtemperatur einer Ligierungsreaktion unterworfen. Dann wurden 2 µl 100 mM ATP, pH 7,5, 6 µl T4DNA-Ligase und 22 µl Wasser zugegeben und die Ligierung über Nacht bei ca. 10°C fortgesetzt.

Die Produkte der Ligierungsreaktion wurden dann mit Xba I und HindIII unter Erhalt einzelner synthetischer Gensegmente mit einer Gesamtlänge von 204 bp geschnitten, diese umfaßten eine offene XbaI-Stelle, eine ribosomale Bindungsstelle, einen Initiationscodon ATG, die Aprotinin-Kodierungssequenz, einen Terminationscodon TAG und eine offene HindIII-Stelle.

Beispiel 3

Konstruktion rekombinanter Plasmide

Die Konstruktionsvektor-DNA (Fig. 2a, b) wurde mit XbaI und HindIII (Maniatis) gespalten, mit synthetischen Gensegmenten, die wie in Beispiel 2 beschreiben, hergestellt worden waren, vermischt und über Nacht ligiert. Die Probe wurde dann zum Transformieren von E. coli Su3 [lac-pro]Δmet⁻ $arg_{am}$ supF thi⁻) nach der Methode von Hahnahan (D. Hahnahan, J. Mol. Biol., 1983, 166, 557-580) verwandt. Die Zellen wurden auf vollmedium-Platten ausplattiert, die 10 µg Tetracyclin/ml und 0,005 % x-gal (5-Brom-4-Chlor-3indolyl-β-D-galactosid; J. Miler, Experiments in Molecular Genetics, 1972, Cold Spring Harbor Laboratory. Cold Spring Harbor, NY), enthielten und wurden über Nacht bei 37 °C inkubiert. Aus blauen Kolonien wurde die Plasmid-DNA extrahiert und auf die Anwesenheit von Restriktionsenzym-Erkennungsstellen, die im synthetischen Gensegment liegen, untersucht. Zum Schluß wurde die DNA-Sequenz nach der Methode von Maxam und Gilbert bestimmt (A. Maxam & W. Gilbert, in Meth. Enzymol., 1983, 65, 499-560).

Beispiel 4

Konstruktion von Multimeren des Gens

piWiT9L1-DNA, die wie im vorhergehenden Beispiel 3 beschrieben,hergestellt worden war, wurde zur Herstellung von Multimeren des Aprotiningens verwendet. Die Plasmid-DNA wurde mit HindIII und BamHI gespalten, und das kleinere Fragment, das das 3'-Ende des Tetracyclinresistenzgens, den "origin of replication", den Promotor und das Met-Aprotiningen enthält, wurde gereinigt (Maniatis et al. (1982)). Eine zweite Probe der Plasmid-DNA wurde mit SbaI und BamHI gespalten und das größere Fragment, welches wiederum das Met-Aprotiningen, das lac-Z-Gen, das Transkriptions-Terminationssignal und das 5'-Ende des Tetracyclinresistenzgens enthält, wurde wie vorstehend gereinigt. Beide Fragmente werden ligiert,(s. Beispiel 2) in Gegenwart der gereinigten Adaptermoleküle,

```
AGCTAATGAGCGCGC
    TTACTCGCGCGATC
```

und damit wird in E. coli BR17 (siehe Beispiel 5) transformiert. Da das Adaptermolekül vorstehende Enden aufweist, die jeweils in eine HindIII-Stelle und eine XbaI-Stelle passen, ohne daß sie eine dieser Stellen regenerieren, besitzt das resultierende neue Plasmid mit zwei tandemartig wiederkehrenden Aprotiningenen ebenfalls nur eine einzige XbaI-Stelle oberhalb der ribosomalen Bindungsstelle des ersten Aprotiningens und auch nur eine einzige HindIII-Stelle unterhalb vom Terminationscodon des zweiten Met-Aprotiningens. Somit können die gleichen Reaktionen unter Verwendung dieser neuen piWiT9L2-DNA durchgeführt werden, wobei ein anderes Plasmid erhalten wird, welches vier tandemartig wiederkehrende Met-Aprotiningene trägt, wobei jedem seine eigene ribosomale Bindungsstelle vorausgeht.

Vor einer dritten Duplizierung der nun vier Aprotiningene wurde das 3Kb EcoRI-Fragment aus dem Plasmid deletiert, um dessen Gesamtgröße zu verringern (Fig. 3). Nach der dritten Verdopplung erhielten wir zufällig ein Plasmid mit sieben Aprotiningenen anstelle von acht. Plasmide mit ungeraden Nummern von Genen findet man gewöhnlich unter den Produkten einer derartigen Ligierungsreaktion mit geringer

Häufigkeit. Wir nehmen an, daß ihre Bildung auf eine teilweise Denaturierung der DNA-Fragmente während des SchmeLzens der "Seaplaque® -Agarose (Maniatis et al.(I982)) im Verlauf des Reinigungsverfahrens zurückzufÜhren ist. Wir konnten keine Abweichung von einer gegebenen Genanzahl nach dem weiteren Wachstum dieser Plasmide in E. coli BR17 feststellen.

Somit ergab die vierte Verdopplung, die wie vorstehend beschrieben durchgeführt wurde, ein Plasmid mit vierzehn Aprotiningenen,˙ die in einem künstlichen Operon zusammengesetzt waren, das in eine polycistronische mRNA transkribiert werden konnte, die dann in 14 getrennte identische Aprotininpeptide übersetzt wurde.

Beisp1el 5

Konstruktion von Expressionsplasmiden

Die Bedeutung des 5'-nicht-translatierten Abschnittes der mRNA, der sogenannten Leader-Sequenz der mRNA, auf die Translationsrate des Gens oder der Gene, die auf dieser mRNA kodieren, ist noch nicht vollständig erklärbar. Wir untersuchten mehrere dieser Leader-Sequenzen auf ihre Translationswirksamkeit und erhielten mit verschiedenen Genen verschiedene Ergebnisse. Eine Leader-Sequenz, mit der mit unserem synthetischen Aprotiningen,die besten Ergebnisse erhalten wurden, ist die Sequenz, die im Expressionsvektor piWiT11 vorliegt (Fig. 4a, b).

Ein Fragment, welches 14 identische Aprotiningene umfaßt, wobei jedes seine eigene ribosome Bindungsstelle enthält, wurde aus dem Konstruktionsplasmid mit XbaI und EcoRI herausgeschnitten und zwischen die XbaI-und EcoRI-Stellen des Polylinkers des Expressionsvektors piWiT11 insertiert. Fig. 5b zeigt, daß ein Hexapeptid codiert von der mRNA, zusätzlich zu den 14 Aprotininpeptiden,translatiert wird. Es wurde berichtet, daß solche kleinen Peptide die Translation des folgenden Gens bzw. der folgenden Gene verstärken (B.E. Schoner et al, Proc. Natl. Acad. Sci. USA, 1984, 81, 5403-5407).

Beispiel 6

Konstruktion des E. coli Wirtstammes BR17

E. coli B1.8 wurde in Berkeley isoliert. Sein Phänotyp, angegeben mit mal⁻ und $\lambda^R$ wurde bestätigt. Dieser Stamm wurde den folgenden Verfahren unterworfen:
1 ml gesättigte Kultur wurde 1 min in einer Eppendorf-Zentrifuge zentrifugiert, die pelettierten Zellen wurden in 1 ml 0,9 % NaCl-Lösung resuspendiert. 0,2 ml dieser Suspension wurden auf einer Minimal-Glukose-Platte, die Thymin bis zu einer Endkonzentration von 0,05 % und Trimethoprim bis zu eienr Endkonzentration von 0,001 %, enthielt, ausplattiert, um die thyA-Mutanten zu selektieren. Eine dieser Kolonien, die auf dieser Platte nach 30 h Inkubation bei 37°C gewachsen war, wurde auf einer weiteren Minimal-Glukose-Agar-Platte mit Thymin und Trimethoprim angezogen und als B13 bezeichnet. Aus dieser Platte wurde eine einzige Kolonie ausgewählt und mit E. coli RZ423, einem recA-defizienten Hfr-Stamm, der ebenfalls $lac^-$gepaart. Diese sexuell sich vereinigenden E. colis wurde auf Minimal-Lactose-Platten ausgestrichen. Die weiblichen B13-Stämme konnte auf diesen Platten aufgrund ihrer Thymin-Auxotrophie nicht wachsen. Die männlichen RZ423-Stämme konnten Lactose nicht als Kohlenstoffquelle verwerten. Somit konnten nur solche B13-Zellen wachsen, die - nach der Paarung mit RZ423 - stabil den Teil des RZ423-Chromosoms inkorporiert hatten, der für ein funktionelles thyA-Gen kodiert. 24 solche Kolonien wurden gereinigt und einzeln auf die Charakteristika $mal^-$ (auf EMBmal-Platten) und $recA^-$ (durch Vergleich des Wachstums in Gegenwart von Methylmethansulfonat) untersucht. Sämtliche der getesteten Kolonien waren $mal^-$ ; auf diese Weise wurde sichergestellt, daß sie nicht zufälligerweise $lac^+$-Revertanden von RZ423 darstellten; drei davon zeigten ein reduziertes Wachstum in Gegenwart von Methylmethansulfonat. was darauf hinwies, daß diese Bakterien das $recA^-$-Allel aus RZ423 gleichzeitig mit dem damit eng verbundenen Wildtyp-thyA-Gen inkorporiert hatten. Sie verfügten nicht über den Hfr-Genotyp, da mit ihnen nicht der männlich-spezifische Bakteriophage M13 auszuplattieren war.

Einer der drei Klone wurde als Wirtstamm für die Expressionsplasmide, die die Inhibitorgene aufweisen, verwendet. Er wurde als E. coli BR17 bezeichnet und bei der Deutschen Sammlung für Mikroorganismen. Göttigen. unter der Nummer DMS 3684 hinterlegt.

Beispiel 7

Herstellung von Met-Aprotinin

Lösungen

Puffer A, pH 8,2
50 mMol Tris-HCl
1 mMol EDTA
eingestellt auf pH 8,2

Puffer B, pH 8,2
8 M Harnstoff
1 mMol Bis-(2-hydroxyethyl)-disulfid
1 mMol 2-Mercaptoethanol
in Puffer A;

Puffer C, pH 8,2
1 mMol Bis-(2-Hydroxyethyl-disulfid)
1 mMol 2-Mercaptoethanol
in Puffer A;

Puffer D, pH 8,2
0,6 Mol Natriumchlorid in Puffer A.

Für Herstellungszwecke wurden 5 l einer E. coli Übernachtkultur des Stammes BR17 piWi T10 wL1,15 min mit 8000 Upm zentrifugiert. Das Zellpellet mit ca. 10 g Gewicht wurde in 25 ml 0,1 M Tris.HCl, 0,001 M EDTA und 0,1 mg Lysozym/ml resuspendiert. Die Zellen wurden mit einer "french press" lysiert.

Das Zellysat wurde 20 min bei 20,000 Upm zentrifugiert. Der Überstand wurde verworfen. Das Pellet wurde zweimal mit 20 ml Puffer gewaschen.

Das Pellet wurde in 10 ml Puffer B gelöst. Die Lösung wurde 1 h bei 50°C unter einer Stickstoffatmosphäre reduziert. Die Lösung wurde dann auf eine Econo-Säule (25 $\times$ 100 mm), die mit ca. 10 ml CM-Sepharose Fast Flow $^{R}$gefüllt war, aufgebracht. Die Säule war mit Puffer B equilibriert worden. Die Säule wurde mit Puffer B gewaschen, bis die Grundlinie stabil war. In einer ersten linearen Gradientenelution wurde die Säule mit 100 ml Puffer B und 100 ml Puffer C eluiert. Vor Aufbringen des zweiten linearen Elutionsgradienten wurde die Säule mit Puffer A gewaschen, bis die Grundlinie stabil war.

Der zweite Gradient wurde aus 100 ml Puffer A und 100 ml Puffer D gebildet. Die Peakfraktionen wurden auf ihre Trypsin-inhibierende Aktivität getestet; außerdem wurde ein ELISA und ein Western-Blot durchgeführt.

In einer Reihe von Experimenten betrug die mit verschiedenen Testen ermittelte Ausbeute 5 bis 20 mg.

Reinigung von Met-Aprotinin durch Chromatographie auf einer Trypsin-Sepharose-Säule:

5 mg renaturiertes Met-Aprotinin wurden in 1 ml 0,1 M Tris-Puffer, pH 6,5, gelöst und auf eine Econo-Säule, die mit 30 ml Trypsin-Sepharose gefüllt war, aufgebracht.

Die Säule war zuvor mit 0,1 M Tris-Puffer, pH 6,5, equilibriert worden. Die Säule wurde dann mit 0,1 M Tris-Puffer, pH 6,5, bis zum Erhalt einer stabilen Grundlinie gewaschen.

Die Säule wurde mit fünf Volumen 0,2 M Na-Acetat-Puffer, pH 4, und dann mit fünf Volumen 0,2 M Essigsäure/HCl, pH 1,8, gewaschen.

Die verschiedenen Fraktionen wurden neutralisiert und ihre Aktivitäten mit Hilfe eines Trypsin-Inhibierungsassays bestimmt.

Die aktiven Fraktionen wurden vereinigt und dann durch Dialyse gegen destilliertes Wasser entsalzt. Der erhaltene Inhibitor wurde durch N-terminale Sequenzierung und Aminosäureanalyse charakterisiert.

Beispiel 8

Herstellung von Met-Ile-15-Aprotinin

Die Fermentation von mit Plasmid piWi T10 Wi7 transformierten E. coli und die Renaturierung von Met-Ile-15-Aprotinin erfolgte nach dem gleichen Verfahren, wie dies in Beispiel 7 beschrieben ist, mit der Ausnahme, daß die Aktivität mit Hilfe eines Elastase-Inhibierungsassays anstelle von einem Trypsin-Inhibierungsassay getestet wurde. In einer Reihe von Experimenten betrug die durch verschiedene Tests bestimmte Ausbeute 5 - 10 mg.

Das renaturierte Met-Ile-15-Aprotinin wurde durch Chromatographie auf einer mit polyklonalem Anti-Aprotinin-Antikörper gefüllten Säule weiter gereinigt.

1 mg Met-Ile-15-Aprotinin wurde in 2 ml 0,05 M Phosphatpuffer, 1 M NaCl, pH 7,0, aufgelöst und auf eine Econo-Säule (41 $\times$ 100 mm), die mit 40 ml polyklonalem Anti-Aprotininantikörper-Sepharose gefüllt war, aufgebracht.

Die Säule wurde mit drei Volumen Ausgangspuffer gewaschen. Der Elastaseinhibitor Met-Ile-15-Aprotinin wurde mit Hilfe von 0,2 M KCl/HCl pH 2,2,desorbiert. Die aktiven Fraktionen wurden vereinigt und gegen destilliertes Wasser dialysiert. Der Inhibitor wurde durch Lyophilisierung erhalten (ca. 600 µg Gewicht). 1 nMol des Inhibitors wurde auf den Gasphasen-Sequenator aufgetragen und durch N-terminale Sequenzierung charakterisiert; außerdem wurde 1 nMol zur Aminosäureanalyse verwendet.

Tabelle 1: N-terminale Sequenzierung von Met-Aprotinin und Met-Ile-15-Aprotinin

1. Met-Aprotinin; ca. 1 nMol der Substanz wurde über 20 Zyklen sequenziert.

```
1               5                   10
Met-Arg-Pro-Asp-Phe-Cys-Leu-Glu-Pro-Pro-Tyr-Thr-Gly-Pro-
15              19
Cys-Lys-Ala-Arg-Ile-Ile-
```

2. Met-Ile-15-Aprotinin; ca. 1 nMol der Substanz wurde über 20 Zyklen sequenziert.

```
1               5                   10
Met-Arg-Pro-Asp-Phe-Cys-Leu-Glu-Pro-Pro-Tyr-Thr-Gly-Pro-
15              19
Cys-Ile-Ala-Arg-Ile-Ile-
```

Tabelle 2: Aminosäureanalyse von Met-Aprotinin, Met-Ile-15-Aprotinin und Aprotinin

| Aminosäure | Met-Aprotinin | Met-Ile-15-Aprotinin | Aprotinin |
|---|---|---|---|
| Asp | 5,06 (5) | 5,12 (5) | 4,90 (5) |
| Thr | 2,71 (3) | 2,80 (3) | 2,89 (3) |
| Ser | 0,98 (1) | 0,95 (1) | 0,94 (1) |
| Glu | 3,15 (3) | 3,20 (3) | 3,05 (3) |
| Gly | 5,97 (6) | 5,92 (6) | 6,05 (6) |
| Ala | 6,00 (6) | 6,00 (6) | 6,00 (6) |
| Val | 0,94 (1) | 1,02 (1) | 1,04 (1) |
| Met | 1,60 (2) | 1,76 (2) | 0,95 (1) |
| Ile | 1,36 (2) | 2,45 (3) | 1,29 (2) |
| Leu | 2,02 (2) | 2,08 (2) | 2,10 (2) |
| Thyr | 4,01 (4) | 3,92 (4) | 3,85 (4) |
| Phe | 3,98 (4) | 3,94 (4) | 3,86 (4) |
| Lys | 4,19 (4) | 3,08 (3) | 3,99 (4) |
| Arg | 6,09 (6) | 5,82 (6) | 5,90 (6) |

Die Aminosäuren wurden nach durch "post column"-Derivatisierung mit o-Phthalaldehyd bestimmt. Cys und Pro wurden nicht bestimmt.

Tabelle 3: Vergleich der inhibierenden Wirkung von Aprotinin und Met-Aprotinin. Die Inhibierung der tryptischen Aktivität wurde mit Hilfe von N-Benzoyl-DL-arginin-p-nitroanilid (BANA) gemessen.

| Aprotinin | | Genprodukt | |
|---|---|---|---|
| Inhibitormenge pro Assay | O.D./10 min | Inhibitormenge pro Assay | O.D./10 min |
| 0 ng | 0,444 | 0 ng | 0,466 |
| 100 ng | 0,391 | 68 ng | 0,398 |
| 200 ng | 0,261 | 136 ng | 0,355 |
| 400 ng | 0,073 | 272 ng | 0,268 |
| Steigung: | -,00096 | Steigung: | -,00071 |
| Schneiden mit der Abszisse: | 479 | Schneiden mit der Abszisse: | 642 |
| Korrelations-koeffizient: | .993 | Korrelations-koeffizient: | .994 |

Legenden zu

Fig. 6: SDS-Gelelektrophorese (15 % Polyacrylamid) von E. coli-Proteinen aus E. coli BR 17 [piWi T10 wL1], E. coli BR 17 [piWi T10 wi7] und E. coli BR 17 [piWi T10w]

1. piWi T10 wi7,$10^9$-Zellen;
2. piWi T10 wL1, $10^9$-Zellen;
3. lösliche Proteine piWi T10 wi7;
4. lösliche Proteine piWi T10 wL1;
5. lösliche Proteine piWi T10w;
6. piWi T10w, 2 × $10^8$-Zellen;
7. piWi T10 wi7, 2 × $10^8$ -Zellen;
8. piWi T10 wL1, 2 x $10^8$-Zellen.

Fig. 7: SDS-Gelelektrophorese (10 - 20 % Polyacrylamid) der Proteine aus E. coli-Stamm BR 17 piWi T10 wL1.

Fig. 9: Durch Renaturierung und Trypsin-Sepharose-Chromatographie erhaltenes rekombinantes Aprotinin wurde einer Elektrophorese auf einem Na-DodS$O_4$ Gradienten (10 - 20 %) Polyacrylamidgel unterworfen und dann ein Western-Blot nach Towbin durchgeführt.

Bahn 1: Aprotinin;

Bahn 2: rekombinantes Aprotinin nach Renaturierung;

Bahn 3: rekombinantes Aprotinin nach Trypsin-Sepharose-Chromatographie .

## Ansprüche

1. Mikrobiell hergestelltes Met-Aprotinin und Met-Aprotinin-Homologe.

2. Mikrobiell hergestelltes Met-Aprotinin, welches in Position 15 durch eine natürlich vorkommende Aminosäure substituiert ist.

3. Mikrobiell hergestelltes Met-Aprotinin, welches in Position 15 durch Arg, Val, Thr, Ile, Leu, Phe, Gly, Ser, Met, Trp, Tyr oder Ala substituiert ist.

4. Mikrobiell hergestelltes Met-Aprotinin nach einem oder mehreren der Ansprüche 1 bis 3, welches in Position 52 durch Glu, Leu, Val, Thr oder Ser substituiert ist.

5. DNA der folgenden Sequenz:

```
        1                   5                       10
met arg pro asp phe cys leu glu pro pro tyr thr gly pro
ATG AGA CCA GAT TTC TGC CTC GAG CCG CCG TAC ACT GGG CCC
TAC TCT GGT CTA AAG ACG GAG CTC GGC GGC ATG ATG CCC GGG
                        XhoI                    ApaI

    15              20                  25
cys lys ala arg ile ile arg tyr phe tyr asN ala lys
TGC AAA GCT CGT ATC ATC CGT TAC TTC TAC AAT GCA AAG
ACG TTT CGA GCA TAG TAG GCA ATG AAG ATG TTA CGT TTC

            30                  35
ala gly leu cys glN thr phe val tyr gly gly cys arg
GCA GGC CTG TGT CAG ACC TTC GTA TAC GGC GGC TGC AGA
CGT CCG GAC ACA GTC TGG AAG CAT ATG CCG CCG ACG TCT
    StuI                        AccI        PstI

40                  45                  50
ala lys arg asN asN phe lys ser ala glu asp
GCT AAG CGT AAC AAC TTC AAA TCC GCG GAA GAC
CGA TTC GCA TTG TTG AAG TTT AGG CGC CTT CTG
                                SstII

                55
cys met arg thr cys gly gly ala
TGC ATG CGT ACT TGC GGT GGT GCT TAG
ACG TAC GCA TGA ACG CCA CCA CGA ATC
SphI
```

und funktionelle Äquivalente derselben.

6. DNA Sequenzen aus der Gruppe

a) RBS

```
CTA GAT TAAAGGAGTTATCTT
         TA ATTTCCTCAATAGAA
```

b)

```
GCA
CGTTCGA
```

c)

```
AGCTAATGAGCGCGC
     TTACTCGCGCGGATC
```

d)

```
                        -35                    Promotor -10
CTCGAGATAAAAAATTTATTTGCTTTCAGGTACAATTCTTGATATAATATTATC
GAGCTCTATTTTTTAAATAAACGAAAGTCCATGTTAAGTTCAATATTATAATAG
XhoI
```

e)

```
mRNA            lac    Operator
ATCTAGCAAATTGTGAGCGGATAACAATT
TAGATCGTTTAACACTCGCCTATTGTTAA
```

```
                                RBS
TGCACACAGCTAGATAATAAAAATTTAAGGGAT
ACGTGTGTCGATCTATTATTTTTAAATTCCCTA
                                  EcoRV
```

f)

```
                        -35                    Promotor -10
CTCGAGATAAAAAATTTATTTGCTTTCAGGTACAATTCTTGATATAATATTATC
GAGCTCTATTTTTTAAATAAACGAAAGTCCATGTTAAGTTCAATATTATAATAG
XhoI
```

```
mRNA            lac    Operator                                       RBS
ATCTAGCAAATTGTGAGCGGATAACAATTTGCACACAGCTAGATAATAAAAATTTAAGGGAT
TAGATCGTTTAACACTCGCCTATTGTTAAACGTGTGTCGATCTATTATTTTTAAATTCCCTA
                                                                 EcoRV
```

RBS

GCAAGCTAATGAGCGCGCCTAGATTAAAGGAGTTATCTT
CGTTCGATTACTCGCGCGGATCTAATTTCCTCAATAGAA

BssHII

RBS

AAGGGATATCTT ATG ACA AGC TTT CTA GAT
TTCCCTATAGAA TAC TGT TCG AAA GAT CTA

EcoRV HindIII XbaI

sowie deren funktionelle Äquivalente.

7. Plasmide enthaltend eine oder mehrere Sequenzen der DNA aus Anspruch 6.

8. E. coli BR17 Mikroorganismen, mit den DSM-Hinterlegungsnummern DSM 3685 und DSM 3686.

9. Pharmazeutische Zubereitung, **dadurch gekennzeichnet,** daß sie mikrobiell hergestelltes Met-Aprotinin und/oder Met-Aprotinin-Homologe umfaßt.

10. Verwendung von mikrobiell hergestelltem Met-Aprotinin und Met-Aprotinin-Homologen bei der Herstellung von Pharmazeutika.

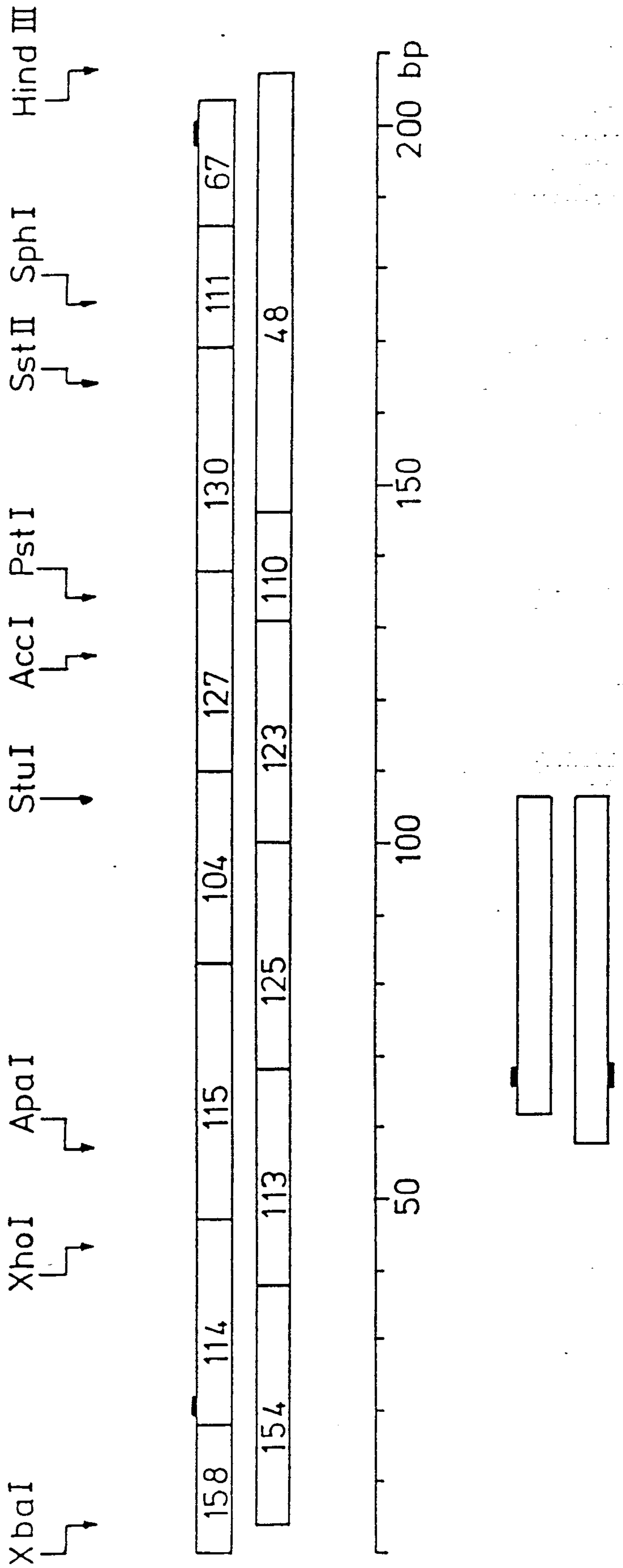
XbaI
XhoI
ApaI
StuI
AccI
PstI
SstII
SphI
HindIII
158
114
115
104
127
130
111
67
154
113
125
123
110
48
50
100
150
200 bp

FIG. 1a

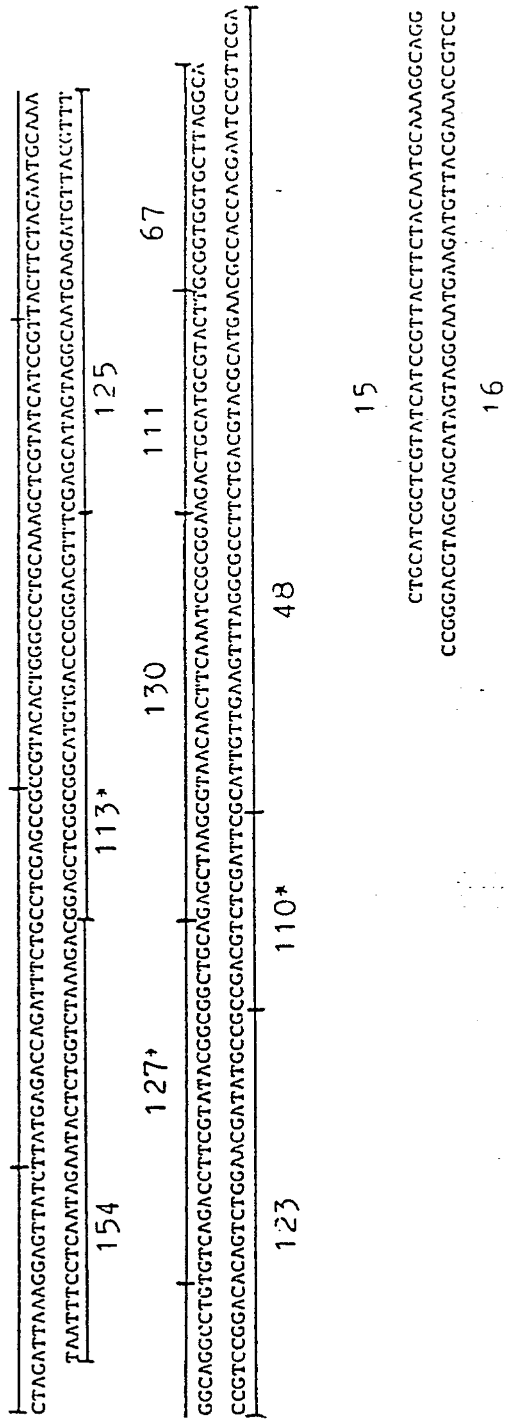


FIG. 1b

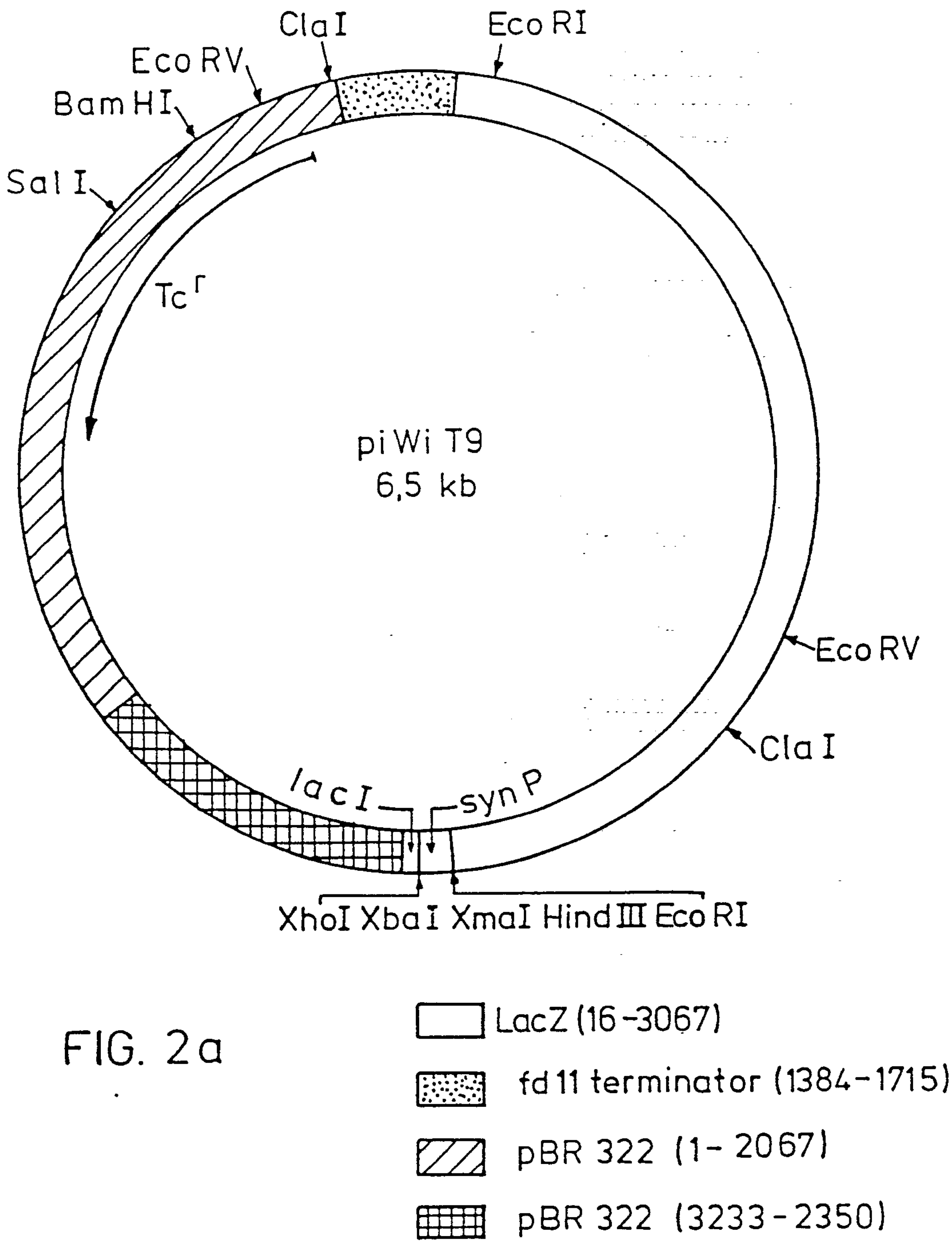
Cla I
Eco RI
Eco RV
Bam HI
Sal I
Tc r
pi Wi T9
6,5 kb
Eco RV
Cla I
lac I
syn P
XhoI XbaI XmaI Hind III Eco RI
LacZ (16-3067)
fd 11 terminator (1384-1715)
pBR 322 (1-2067)
pBR 322 (3233-2350)

FIG. 2a

```
.....pBR322 DNA 2370   2360        codons 322-341 of lacI                          synthetic DNA
.....GCGAGTCAGTGAGCGAGGAAGCGGAAGAGCGCCCAATACCGCAAACCGCCTCTCCCCGCGCCCTCGAGATAAAAATTTATTTGCTTTCAGGTAC
.....CGCTCAGTCACTCGCTCCTTCGCCTTCTCGCGGGTTATGGCGTTTTGGCGGAGAGGGGCGCGGGAGCTCTATTTTTAAATAAACGAAAGACCATG
                                 HaeII                                XhoI

                                                                   6           10          14
                                                                 ser leu ala val val leu glN arg arg ........
AATTCTTGATATAATATTATCATCTAGATTAAGGACCCGGGAAGCTTTCCGGGCAATTCA CTG GCC GTC GTT TTA CAA CGT CGT ........
TTAAGAACTATATTATAATAGTAGATCTAATTCCTGGGCCCTTCGAAAGGCCCCTTAAGT GAC CGG CAG CAA AAT GTT GCA GCA ........
                       XbaI          XmaI  HindIII     EcoRI
                                     SmaI                                          lacZ DNA ........

                 1010                                     1020        1023
    glu phe glN leu ser ala gly arg tyr his tyr glN leu val trp cys glN lys                      +4bp
.....CAA TTC CAG CTG AGC GCC GGT CGC TAC CAT TAC CAG TTG GTC TGG TGT CAA AAA TAATAATAACCGGCAAGGGGATCC
.....CTT AAG GTC GAC ACG CGG CCA GCG ATG GTA ATG GTC AAC CAG ACC ACA GTT TTT ATTATTATTGGCCGTTCCCCTAGG
    EcoRI   PvuII                     lacZ DNA

   1390      1400                                                               1700    1710
ATCCCGCAAAAGCGGCCTTTGACTCCCT.....  transcription termination sequence  .....GGAAAGACGACAAAACTTTAGATC
TAGGGCGTTTTCGCCGGAAACTGAGGGA.....                                      .....CCTTTCTGCTGTTTTGAAATCTAG
                                           fd11 DNA

  +4bp            10          20           +4bp        40
CGGGGAATTAATTCTCATGTTTGACAGCTTATCATCGATAAGCTAGCTTTAATGCGGTA.....   tetracycline resistance gene
GCCCCTTAATTAAGAGTACAAACTGTCGAATAGTAGCTATTCGATCGAAATTACGCCAT.....
pBR322 DNA                             ClaI     NheI

        2050       2060       2220       2210       2200
.....CCACGCTGATGAGCTTTACCGCAGAAAAGGATCTAGGTGAAGATCCTTTTTGAT.....   origine of replication of pBR 322
.....GGTGCGACTACTCGAAATGGCGTCTTTTCCTAGATCCACTTCTAGGAAAAACTA.....
.....pBR322 DNA      1/2PvuII | 1/2AhaIII
```

FIG. 2b

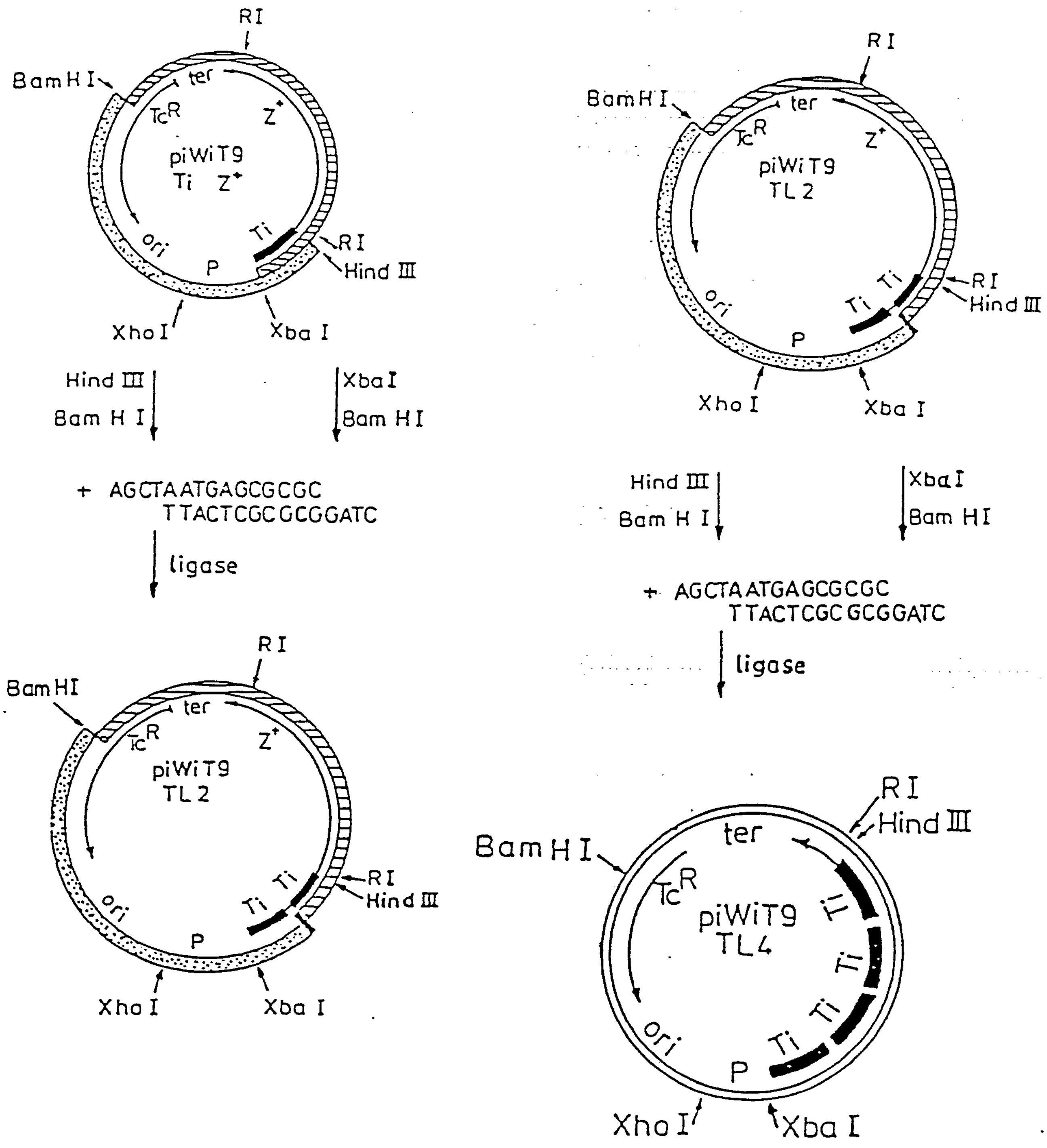
RI
BamHI
ter
TcR
Z+
piWiT9
Ti Z+
ori
Ti
RI
Hind III
P
XhoI
Xba I
Hind III
Bam H I
XbaI
Bam HI
+ AGCTAATGAGCGCGC
TTACTCGCGCGGATC
ligase
piWiT9
TL2
piWiT9
TL4

FIG. 3

FIG. 4a

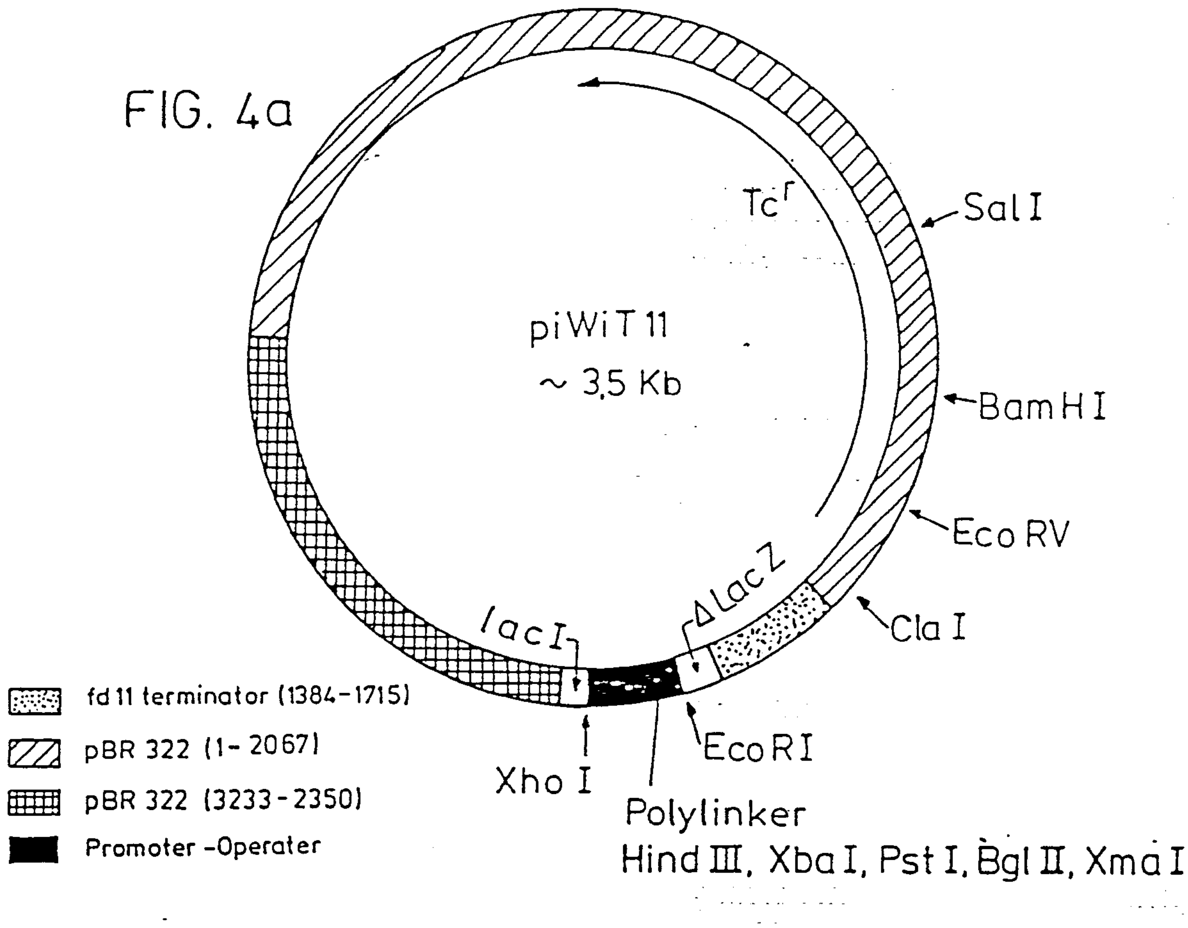
Tc^r
piWiT 11
~ 3,5 Kb
Sal I
BamH I
Eco RV
Cla I
ΔLacZ
lacI
EcoR I
Xho I
Polylinker
Hind III, Xba I, Pst I, Bgl II, Xma I
fd 11 terminator (1384-1715)
pBR 322 (1-2067)
pBR 322 (3233-2350)
Promoter -Operater

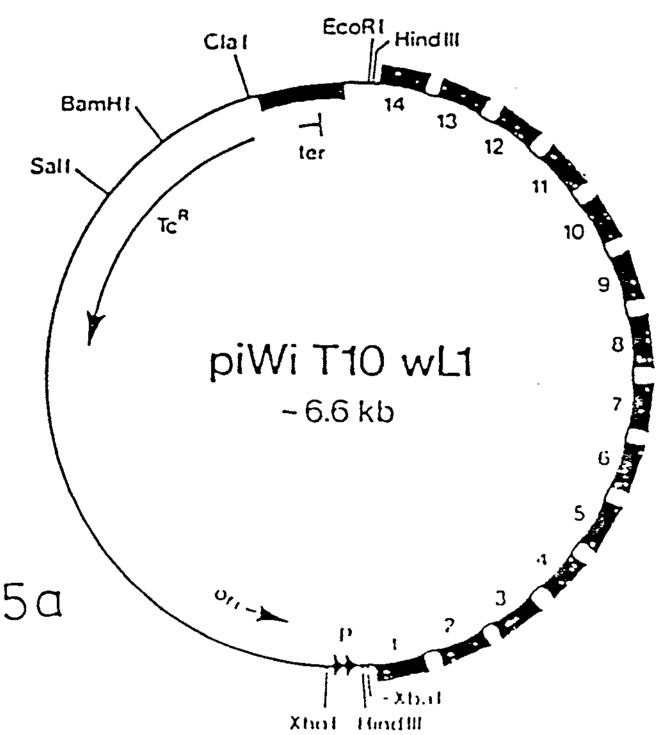
ClaI
EcoRI
HindIII
BamHI
SalI
ter
Tc^R
piWi T10 wL1
~6.6 kb
14
13
12
11
10
9
8
7
6
5
3
2
1
XhoI
HindIII
XbaI

FIG. 5a

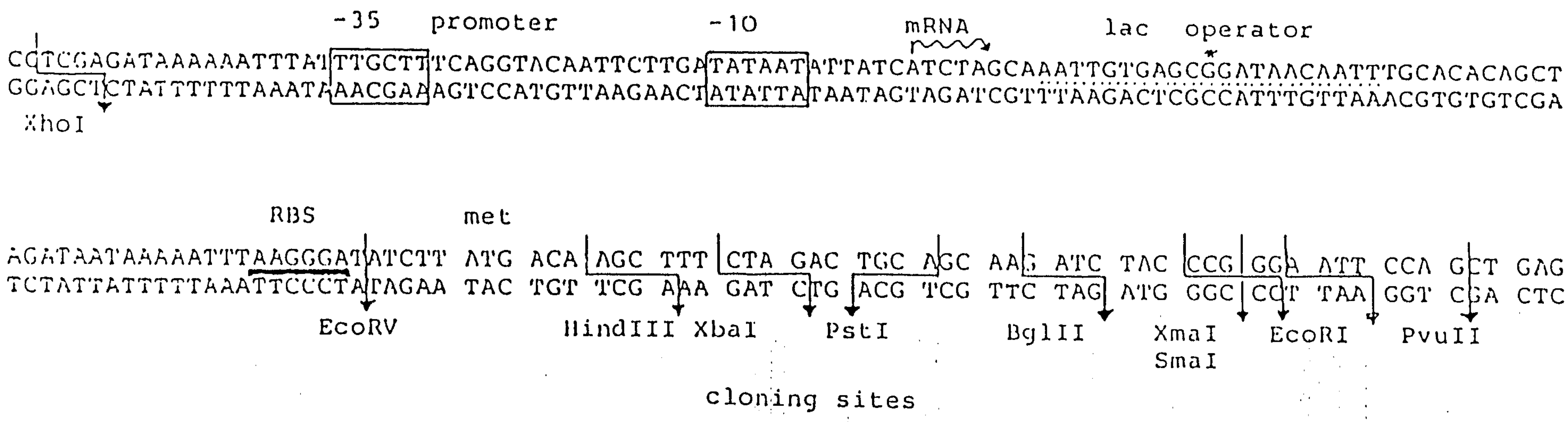
-35
promoter
-10
mRNA
lac operator
CCTCGAGATAAAAAATTTATTTGCTTTCAGGTACAATTCTTGATATAATATTATCATCTAGCAAATTGTGAGCGGATAACAATTTGCACACAGCT
GGAGCTCTATTTTTTAAATAAACGAAAGTCCATGTTAAGAACTATATTATAATAGTAGATCGTTTAACACTCGCCTATTGTTAAACGTGTGTCGA
XhoI
RBS
met
AGATAATAAAAATTTAAGGGATATCTT ATG ACA AGC TTT CTA GAC TGC AGC AAG ATC TAC CCG GGA ATT CCA GCT GAG
TCTATTATTTTTAAATTCCCTATAGAA TAC TGT TCG AAA GAT CTG ACG TCG TTC TAG ATG GGC CCT TAA GGT CGA CTC
EcoRV
HindIII
XbaI
PstI
BglII
XmaI
SmaI
EcoRI
PvuII
cloning sites

FIG. 4b

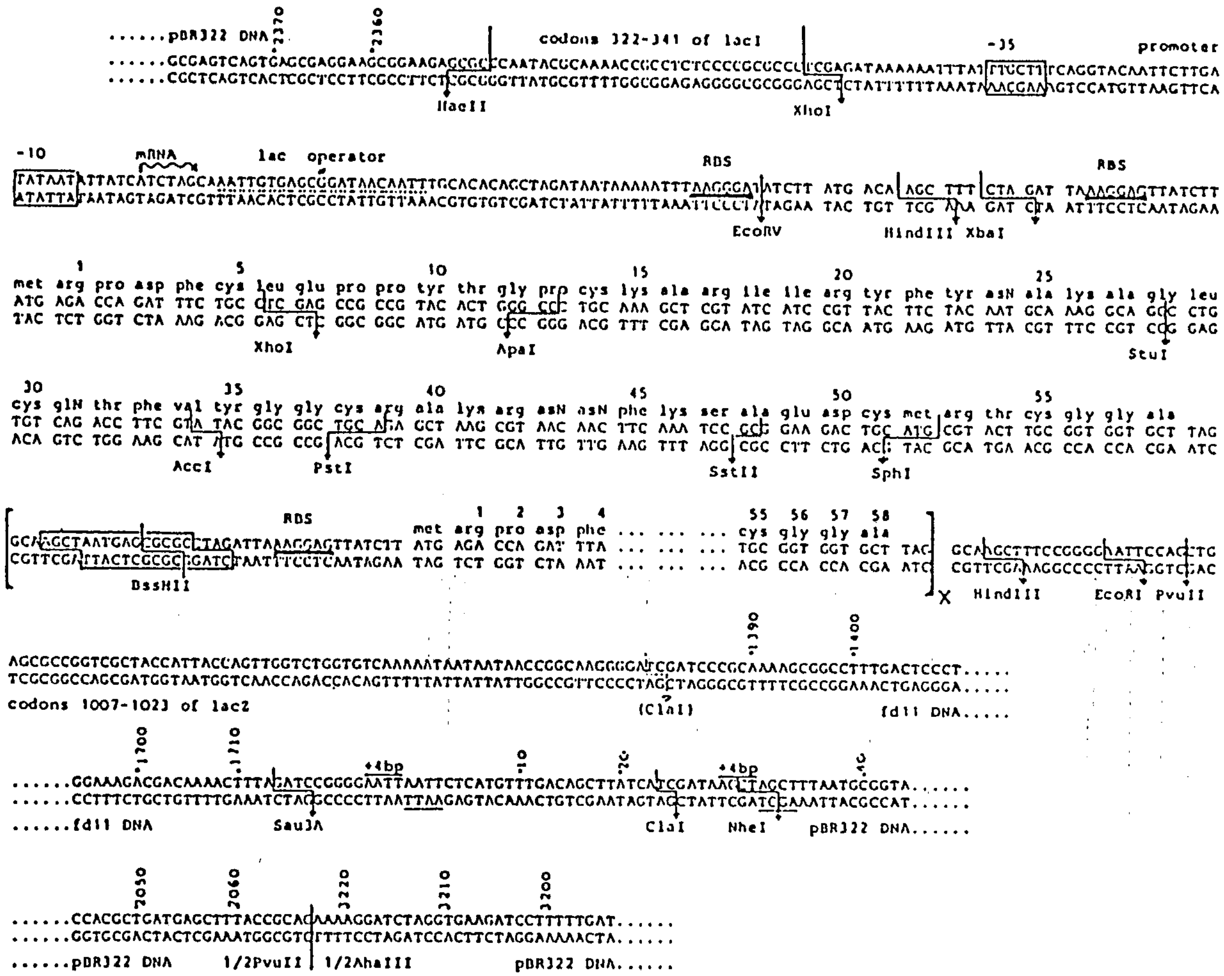
......pBR322 DNA 2370 2360 codons 322-341 of lacI -35 promoter
......GCGAGTCAGTGAGCGAGGAAGCGGAAGAGCGCCCAATACGCAAACCGCCTCTCCCCGCGCGTTGGCCGATTCGAGATAAAAAATTTATTTGCTTTCAGGTACAATTCTTGA
......CGCTCAGTCACTCGCTCCTTCGCCTTCTCGCGGGTTATGCGTTTGGCGGAGAGGGGCGCGGAGCTCTATTTTTTAAATAAACGAAAGTCCATGTTAAGTTCA
HaeII XhoI
-10 mRNA lac operator RBS RBS
TATAATATTATCATCTAGCAAATTGTGAGCGGATAACAATTTGCACACAGCTAGATAATAAAAATTTAAGGGAATCTT ATG ACA AGC TTT CTA GAT TAAAGGAGTTATCTT
ATATTATAATAGTAGATCGTTTAACACTCGCCTATTGTTAAACGTGTGTCGATCTATTATTTTTAAATTCCCTTAGAA TAC TGT TCG AAA GAT CTA ATTTCCTCAATAGAA
EcoRV HindIII XbaI
1 5 10 15 20 25
met arg pro asp phe cys leu glu pro pro tyr thr gly pro cys lys ala arg ile ile arg tyr phe tyr asN ala lys ala gly leu
ATG AGA CCA GAT TTC TGC CTC GAG CCG CCG TAC ACT GGG CCC TGC AAA GCT CGT ATC ATC CGT TAC TTC TAC AAT GCA AAG GCA GGC CTG
TAC TCT GGT CTA AAG ACG GAG CTC GGC GGC ATG ATG CCC GGG ACG TTT CGA GCA TAG TAG GCA ATG AAG ATG TTA CGT TTC CGT CCG GAC
XhoI ApaI StuI
30 35 40 45 50 55
cys glN thr phe val tyr gly gly cys arg ala lys arg asN asN phe lys ser ala glu asp cys met arg thr cys gly gly ala
TGT CAG ACC TTC GTA TAC GGC GGC TGC AGA GCT AAG CGT AAC AAC TTC AAA TCC GCG GAA GAC TGC ATG CGT ACT TGC GGT GGT GCT TAG
ACA GTC TGG AAG CAT ATG CCG CCG ACG TCT CGA TTC GCA TTG TTG AAG TTT AGG CGC CTT CTG ACG TAC GCA TGA ACG CCA CCA CGA ATC
AccI PstI SstII SphI
RBS 1 2 3 4 55 56 57 58
GCAAGCTAATGACGCGCGCTAGATTAAAGGAGTTATCTT ATG AGA CCA GAT TTA ... ... ... cys gly gly ala
GCAAGCTAATGACGCGCGCTAGATTAAAGGAGTTATCTT ATG AGA CCA GAT TTA ... ... ... TGC GGT GGT GCT TAC GCAAGCTTTCCGGGGAATTCCAGCTG
CGTTCGATTACTCGCGCGCGATCTAATTTCCTCAATAGAA TAC TCT GGT CTA AAT ... ... ... ACG CCA CCA CGA ATC CGTTCGAAAGGCCCCTTAAGGTCGAC
BssHII X HindIII EcoRI PvuII
1390 1400
AGCGCCGGTCGCTACCATTACCAGTTGGTCTGGTGTCAAAAATAATAATAACCGGCAAGGGGATCGATCCCGCAAAAGCGGCCTTTGACTCCCT.....
TCGCGGCCAGCGATGGTAATGGTCAACCAGACCACAGTTTTTATTATTATTGGCCGTTCCCCTAGCTAGGGCGTTTTCGCCGGAAACTGAGGGA.....
codons 1007-1023 of lacZ (ClaI) fd11 DNA.....
1700 1710 +4bp +4bp
......GGAAAGACGACAAAACTTTAGATCCGGGGAATTAATTCTCATGTTTGACAGCTTATCATCGATAAGCTAGCTTTAATGCGGTA......
......CCTTTCTGCTGTTTTGAAATCTAGGCCCCTTAATTAAGAGTACAAACTGTCGAATAGTAGCTATTCGATCGAAATTACGCCAT......
......fd11 DNA Sau3A ClaI NheI pBR322 DNA......
2050 2060 2220 2210 2200
......CCACGCTGATGAGCTTTACCGCAGAAAAGGATCTAGGTGAAGATCCTTTTTGAT......
......GGTGCGACTACTCGAAATGGCGTCTTTTCCTAGATCCACTTCTAGGAAAAACTA......
......pBR322 DNA 1/2PvuII 1/2AhaIII pBR322 DNA......

FIG. 5b

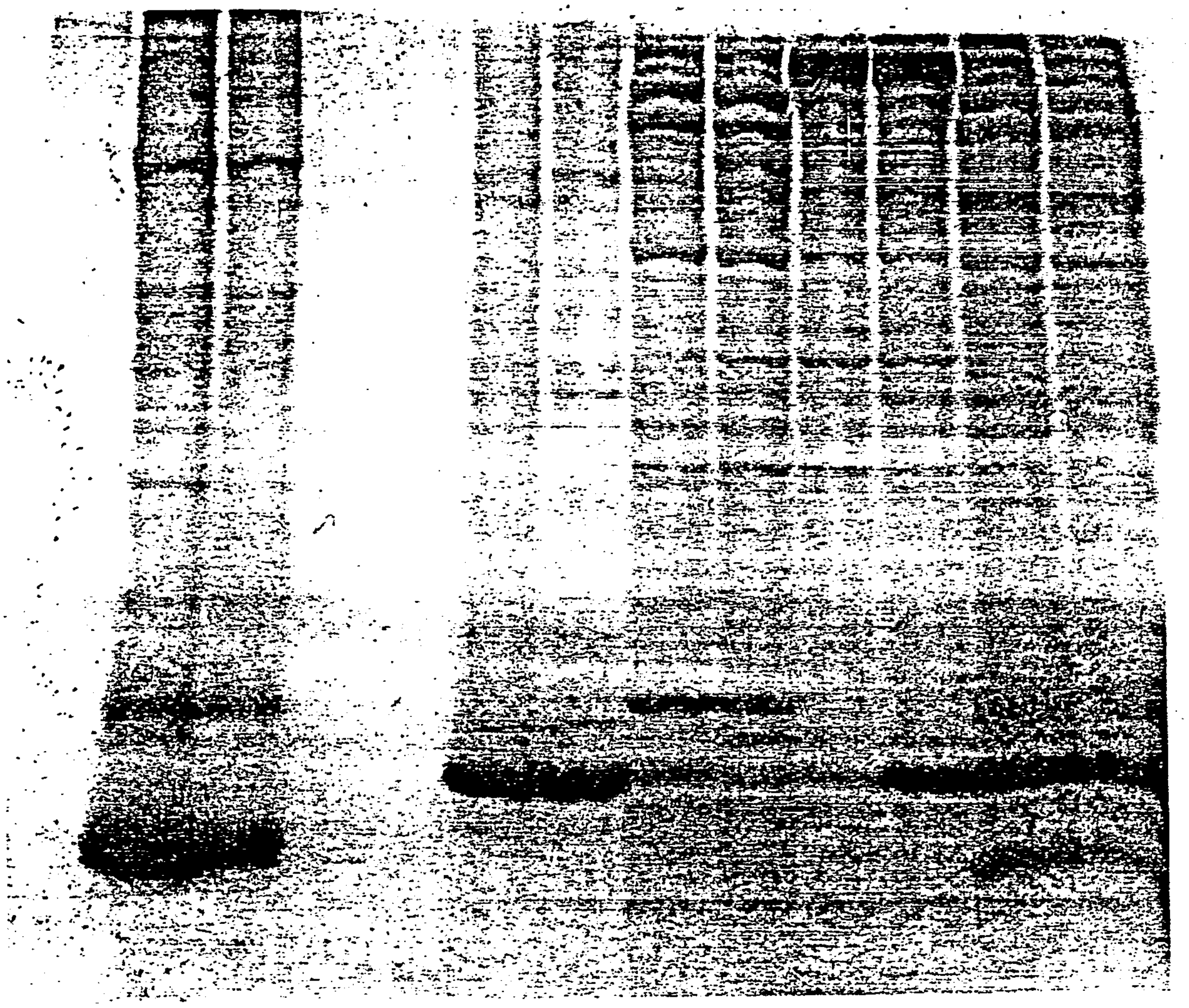

FIG. 6

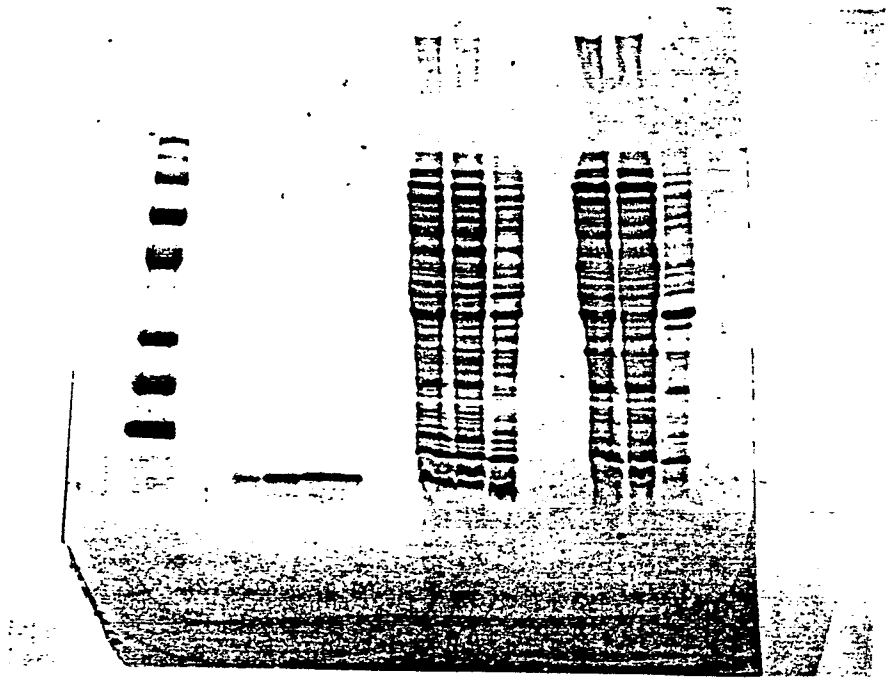

FIG. 7

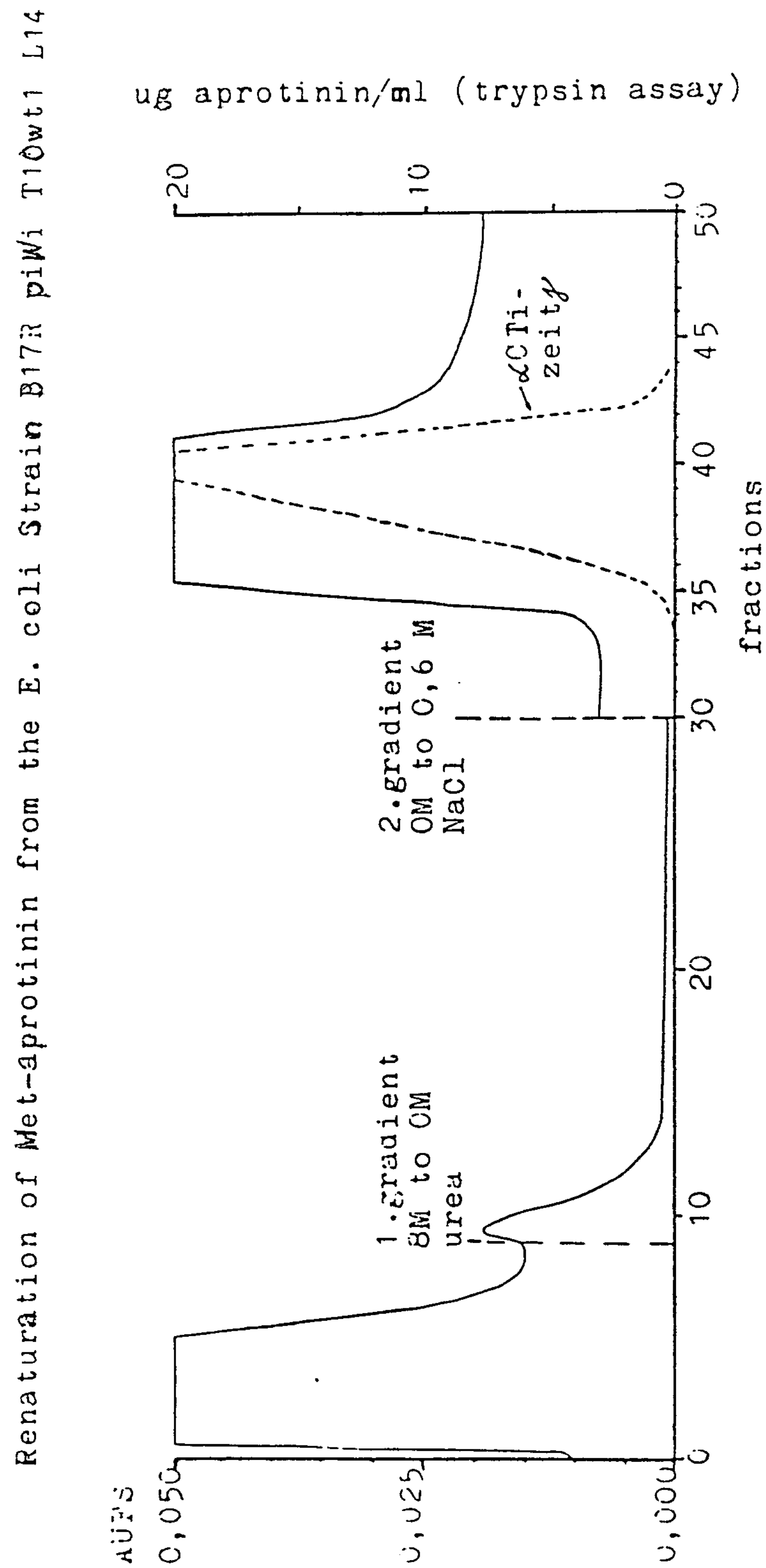

Renaturation of Met-aprotinin from the E. coli Strain B17R piWi T10wt1 L14
ug aprotinin/ml (trypsin assay)
20
10
0
αCTi-zeity
2.gradient 0M to 0,6 M NaCl
1.gradient 8M to 0M urea
AUFS
0,050
0,025
0,000
0
10
20
30
35
40
45
50
fractions


FIG. 8

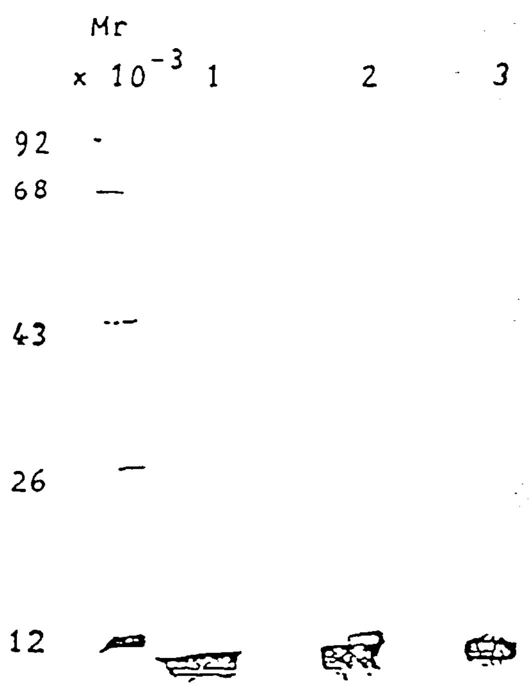


FIG. 9

The renatured Met-aprotinin was additionally purified on a trypsinsepharose column.

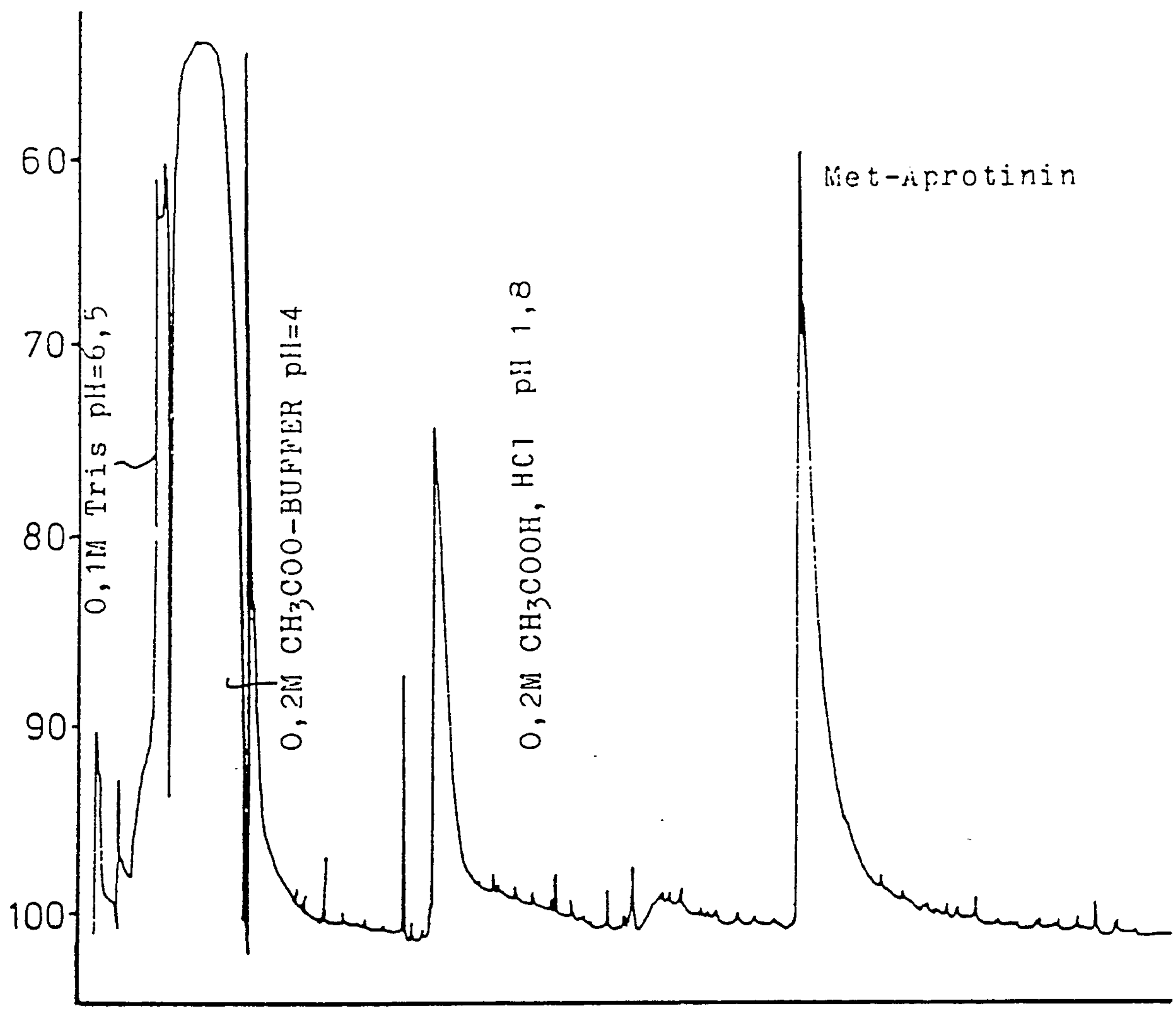


FIG. 10